# NEUE EUROPÄISCHE PATENTSCHRIFT

(11) **EP 2 961 486 B2**
(45) Veröffentlichungstag und Bekanntmachung des Hinweises auf die Entscheidung über den Einspruch: **06.04.2022**
(45) Hinweis auf die Patenterteilung: 21.11.2018
(21) Anmeldenummer: 14702827.8
(22) Anmeldetag: 03.02.2014
(51) Int. Cl.: A61Q 5/10, A61K 8/34, A61K 8/41, A61K 8/49

(54) **MULTITONALE EINSCHRITTFÄRBUNGEN II**
MULTITONAL ONE-STEP PROCESS ON HAIR DYEING
COLORISATION MULTITONALE DE CHEVEUX À UNE ÉTAPPE

(30) Priorität: 27.02.2013 DE 102013203229
(43) Veröffentlichungstag der Anmeldung: 06.01.2016
(73) Patentinhaber: Henkel AG & Co. KGaA, 40589 Düsseldorf (DE)
(72) Erfinder: NEUBA, Constanze, 41516 Grevenbroich (DE); JANßEN, Frank, 51103 Köln (DE)
(86) Internationale Anmeldenummer: PCT/EP2014/052044
(87) Internationale Veröffentlichungsnummer: WO 2014/131580

(56) Entgegenhaltungen:
- DE-A1- 10 037 580
- DE-A1- 19 718 187
- US-A1- 2003 154 562
- US-A1- 2005 000 035

## Beschreibung

Gegenstand der vorliegenden Anmeldung ist ein Verfahren für die Behandlung keratinischer Fasern, welches es erlaubt, in einem Färbeschritt das Haar zu colorieren und gleichzeitig eine multitonale Färbung mit helleren ("highlights") oder dunkleren ("lowlights") Partien (Strähnchen) zu erzeugen.

Im Laufe der Zeit und insbesondere unter Einwirkung von äußeren Einflüssen wie Licht oder atmosphärischen Schadstoffen verliert oder verändert das Haar seine natürliche Farbe und seinen Glanz bzw. Schimmer. Aus diesem Grund finden Haarfärbemittel breite Anwendung, die entweder im Friseurbereich oder durch die Heimanwendung genutzt werden. Für permanente, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten, die unter dem Einfluss von Oxidationsmitteln oder von Luftsauerstoff untereinander die eigentlichen Farbstoffe ausbilden. Die Oxidationsfärbemittel zeichnen sich durch hervorragende, lang anhaltende Färbeergebnisse aus. Für temporäre Färbungen werden üblicherweise Färbe- oder Tönungsmittel verwendet, die als färbende Komponente sogenannte direktziehende Farbstoffe ("Direktzieher") enthalten. Neben der Färbung ist das Aufhellen der eigenen Haarfarbe bzw. das Blondieren der ganz spezielle Wunsch vieler Verbraucher, da eine blonde Haarfarbe als attraktiv und in modischer Hinsicht erstrebenswert betrachtet wird. Sollen Substrate aufgehellt oder gar gebleicht werden, werden die das Substrat färbenden Farbstoffe meist oxidativ unter Einsatz von entsprechenden Oxidationsmitteln, wie Wasserstoffperoxid, entfärbt.

Bei der Haarfärbung - insbesondere bei der Haarfärbung durch die Heimanwendung - tritt das Problem auf, daß natürliche Farbnuancierungen vollständig überdeckt werden, so daß multitonale Färbungen schwer zu realisieren sind.

Um dem Haar ein natürlicheres Aussehen zu verleihen, wird vorgeschlagen, gefärbte Haare durch gezielte Anwendung von Oxidationsmitteln partiell zu entfärben. Die Haarpartien ("Strähnchen"), auf die die Oxidationsmittel aufgetragen werden, bleichen dabei mindestens anteilsweise aus, woraus eine multitonale Haarfarbe resultiert. Die Applikation des Oxidationsmittels erfolgt dabei mit einer Bürste oder einem Pinsel, wobei die nicht zu behandelnden Haare gegebenenfalls mit Aluminiumfolie oder einer sogenannten "Strähnchenhaube" vor der Entfärbung geschützt werden.

Diese Applikationsart löst zwar das Problem der möglichst natürlichen Färbung von Haaren, läßt aber nur das Setzen von "highlights" zu. Für "lowlights", d.h. dunklere Partien müßte nachgefärbt werden. In jedem der Fälle ist ein zeitaufwendiger zweiter Entfärbe- bzw. Färbeschritt vonnöten, der sich an die ursprüngliche Färbung anschließt. Insbesondere bei der Heimanwendung muß daher zunächst das gesamte Haar coloriert werden, bevor die Anwenderin "highlights" oder "lowlights" setzen kann. Von vielen Verbraucherinnen wird dies als zu zeitaufwendig und auch als frustrierend empfunden, da der wesentliche farbverändernde Schritt anfangs erfolgt und in einem zweiten Schritt "korrigiert" wird. Zweistufige oxidative Haarfärbeverfahren, worin zur Steigerung der Färbeleistung zunächst inaktive Färbelösungen auf das Haar verbracht und das Oxidationsmittel in einem nachfolgenden Schritt hinzugefügt wird, sind aus US 2005/000035 und US 2003/154562 bekannt.

Der vorliegenden Erfindung lag die Aufgabe zugrunde, ein Verfahren bereitzustellen, das multitonale Färbungen in einem einzelnen Färbeschritt ermöglicht. Dabei sollte die Färbung des Haares mit der Erzeugung von "highlights" oder "lowlights" einhergehen, damit nach dem Ausspülen des Färbemittels direkt ein Ergebnis sichtbar ist.

Es wurde nun gefunden, daß eine partielle Vorbehandlung von Faserarealen bzw. Strähnchen dazu führt, daß diese Areal bzw. Strähnchen später intensiver oder weniger intensiv angefärbt werden. Durch die Vorpenetration einzelner Faserareale bzw. Strähnchen färbt das unmittelbar darauffolgend angewendete Färbemittel das Haar multitonal, und die Verbraucherin kann direkt nach dem Färbeschriit ein natürliches Färbeergebnis mit "highlights" oder "lowlights" bewundern.

Gegenstand der vorliegenden Erfindung ist in einer ersten Ausführungsform ein Verfahren zum oxidativen Färben von keratinischen Fasern, umfassend die Schritte
A) Applikation eines kosmetischen Mittels (A) auf die Fasern,
B) Einwirkenlassen des Mittels (A) für einen Zeitraum von 30 Sekunden bis 30 Minuten,
C) Applikation eines kosmetischen Mittels (B) auf die keratinischen Fasern, die noch mit dem Mittel (A) beaufschlagt sind,
D) Einwirkenlassen beider Mittel (A) und (B) für einen Zeitraum von 5 bis 45 Minuten,
E) Ausspülen der Mittel (A) und (B),
bei dem das Mittel (A)
(a1) ein oder mehrere Oxidationsfarbstoffvorprodukte vom Entwicklertyp enthält,
(a2) ein oder mehrere Oxidationsfarbstoffvorprodukt vom Kupplertyp enthält,
(a3) einen pH-Wert von 8,0 bis 12,0 besitzt, und - bezogen auf sein Gewicht - weniger als 0.01 Gew.-% Wasserstoffperoxid enthält, und

- das Mittel (B)
   (b1) ein oder mehrere Oxidationsfarbstoffvorprodukte enthält,
   (b2) eine oder mehrere Oxidationsmittel enthält und
   (b3) einen pH-Wert von 8,0 bis 12,0 besitzt.

Im ersten Schritt des erfindungsgemäßen Verfahrens wird ein kosmetisches Mittel (A) auf die Fasern appliziert. Dieses Mittel (A), das nachfolgend auch als Vorbehandlungsmittel oder als Vorpenetrationsmittel bezeichnet wird, wird über einen Zeitraum von 30 Sekunden bis 30 Minuten auf den keratinischen Fasern belassen (Schritt B) des erfindungsgemäßen Verfahrens). Erfindungsgemäß bevorzugte Verfahren sind durch eher kürzere Einwirkzeiten des Vorbehandlungsmittels gekennzeichnet. Besonders bevorzugte erfindungsgemäße Verfahren sind dadurch gekennzeichnet, daß das Mittel (A) im Schritt B) für einen Zeitraum von 30 Sekunden bis 15 Minuten, bevorzugt von 30 Sekunden bis 10 Minuten und besonders bevorzugt von 30 Sekunden bis 5 Minuten auf den Fasern einwirken gelassen wird.

Besonders bevorzugte erfindungsgemäße Verfahren sind weiterhin gekennzeichnet durch das B) Einwirkenlassen des Mittels (A) für einen Zeitraum von 2 bis 10 Minuten bei einer Temperatur von 20 bis 60 °C, bevorzugt von 25 bis 55 °C, weiter bevorzugt von 27 bis 50 °C und ganz besonders bevorzugt von 30 bis 45 °C.

Um multitonale Färbungen zu erzeugen, sollte des Mittel (A) nicht gleichmäßig auf die keratinischen Fasern appliziert werden. Vorzugsweise werden nur einzelne Bereiche, besonders bevorzugt nur einzelne Strähnen mit dem Mittel (A) beaufschlagt. Alternativ kann die Konzentration des Mittels A auf einzelnen Strähnchen variiert werden. Es ist auch möglich, zunächst die gesamten keratinischen Fasern mit dem Mittel (A) gleichmäßig zu beaufschlagen und dann einzelne Bereiche nochmals mit dem Mittel (A) zu behandeln. Auch eine mehrmalige Behandlung einzelner Bereiche/Strähnen mit dem Mittel (A) ist erfindungsgemäß möglich.

Besonders bevorzugte erfindungsgemäße Verfahrens sind dadurch gekennzeichnet, daß die Applikation des kosmetischen Mittels (A) auf die Fasern in Schritt A) nur auf einzelne Strähnchen erfolgt.

Im Anschluß an die Einwirkzeit des Vorbehandlungsmittels werden die keratinischen Fasern nicht ausgespült oder abfrottiert. Vielmehr wird in Schritt C) des erfindungsgemäßen Verfahrens ein kosmetisches Mittel (B) auf die noch mit dem Mittel (A) beaufschlagten Fasern appliziert. Die durch Applikation des Mittels (B) auf den keratinischen Fasern entstandene Mischung aus den Mitteln (A) und (B) wird in Schritt D) des erfindungsgemäßen Verfahrens für einen Zeitraum von 5 bis 45 Minuten Einwirken gelassen.

Erfindungsgemäß bevorzugte Verfahren sind durch eher kürzere Einwirkzeiten der Mischung aus den Mitteln (A) und (B) gekennzeichnet. Besonders bevorzugte erfindungsgemäße Verfahren sind dadurch gekennzeichnet, daß die Mittel (A) und (B) in Schritt D) für einen Zeitraum von 5 bis 30 Minuten, bevorzugt von 5 bis 20 Minuten, besonders bevorzugt von 5 bis 15 Minuten einwirken gelassen werden.

Die letztgenannten Einwirkzeiten beziehen sich dabei auf die Mischung der Mittel (A) und (B). Da das Mittel (A) in Schritt B) des erfindungsgemäßen Verfahrens bereits einwirkte, haben die Fasern insgesamt zu den Inhaltsstoffen des Mittels (A) längeren Kontakt als zu denen des Mittels (B). Wenn das Mittel (A) nur auf einzelne Strähnchen bzw. in einzelnen Areaklen abgewendet wurde, konnten die Inhaltsstoffe des Mittels (A) in diesen Bereichen intensiver einwirken und somit die Wirkung der Inhaltsstoffe des Mittels (B) in diesen Bereichen verstärken oder abschwächen, wodurch eine dunklere oder hellere Färbung dieser Bereiche erzielt wird.

Nach dem Ausspülen der Mittel (A) und (B) in Schritt E) des erfindungsgemäßen Verfahrens erlebt die Verbraucherin unmittelbar eine multinales Farberlebnis, ohne einen weiteren Schritt durchführen zu müssen.

Erfindungsgemäß enthält das Mittel (A) ein oder mehrere Oxidationsfarbstoffvorprodukte vom Entwicklertyp (a1) und ein oder mehrere Oxidationsfarbstoffvorprodukte vom Kupplertyp (a2). Bevorzugte Oxidationsfarbstoffvorprodukte vom Entwicklertyp sind p-Phenylendiaminderivate. Bevorzugte p-Phenylendiamine werden ausgewählt aus einer oder mehrerer Verbindungen der Gruppe p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-3-methyl-(N,N-diethyl)anilin, N,N-Bis-(2-hydroxyethyl)-p-phenylendiamin, 4-N,N-Bis-(2-hydroxyethyl)amino-2-methylanilin, 4-N,N-Bis-(2-hydroxyethyl)-amino-2-chloranilin, 2-(2-Hydroxyethyl)-p-phenylendiamin, 2-(1,2-Dihydroxyethyl)-p-phenylendiamin, 2-Fluor-p-phenylendiamin, 2-Isopropyl-p-phenylendiamin, N-(2-Hydroxypropyl)-p-phenylendiamin, 2-Hydroxymethyl-p-phenylendiamin, N,N-Dimethyl-3-methyl-p-phenylendiamin, N-Ethyl-N-2-hydroxyethyl-p-phenylendiamin, N-(2,3-Dihydroxypropyl)-p-phenylendiamin, N-(4'-Aminophenyl)-p-phenylendiamin, N-Phenyl-p-phenylendiamin, 2-(2-Hydroxyethyloxy)-p-phenylendiamin, 2-Methoxymethyl-p-phenylendiamin, 2-(2-Acetylaminoethyloxy)-p-phenylendiamin, N-(2-Methoxyethyl)-p-phenylendiamin, N-(4-Amino-3-methylphenyl)-N-[3-(1H-imidazol-1-yl)propyl]amin, 5,8-Diaminobenzo-1,4-dioxan sowie ihren physiologisch verträglichen Salzen. Erfindungsgemäß besonders bevorzugte p-Phenylendiaminderivate sind ausgewählt aus mindestens einer Verbindung der Gruppe p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, N-(4-Amino-3-methylphenyl)-N-[3-(1H-imidazol-1-yl)propyl]amin, 2-Methoxymethyl-p-phenylendiamin sowie deren physiologisch verträglichen Salzen. Es kann erfindungsgemäß weiterhin bevorzugt sein, als Entwicklerkomponente Verbindungen einzusetzen, die mindestens zwei aromatische Kerne enthalten, die mit Amino- und/oder Hydroxylgruppen substituiert sind. Bevorzugte zweikernige Entwicklerkomponenten werden insbesondere aus mindestens einer der folgenden Verbindungen ausgewählt: N,N'-Bis-(2-hydroxyethyl)-N,N'-bis-(4'-aminophenyl)-1,3-diaminopropan-2-ol, N,N'-Bis-(2-hydroxyethyl)-N,N'-bis-(4'-aminophenyl)ethylendiamin, N,N'-Bis-(4'-aminophenyl)tetramethylendiamin, N,N'-Bis-(2-hydroxyethyl)-N,N'-bis-(4'-aminophenyl)-tetramethylendiamin, N,N'-Bis-(4-(methylamino)phenyl)tetramethylendiamin, N,N'-Diethyl-N,N'-bis-(4'-amino-3'-methylphenyl)ethylendiamin, Bis-(2-hydroxy-5-aminophenyl)methan, N,N'-Bis-(4'-aminophenyl)-1,4-diazacycloheptan, N,N'-Bis-(2-hydroxy-5-aminobenzyl)piperazin, N-(4'-Aminophenyl)-p-phenylendiamin und 1,10-Bis-(2',5'-diaminophenyl)-1,4,7,10-tetraoxadecan sowie ihre physiologisch verträglichen Salze. Ganz besonders bevorzugte zweikernige Entwicklerkomponenten werden ausgewählt unter N,N'-Bis-(2-hydroxyethyl)-N,N'-bis-(4-aminophenyl)-1,3-diamino-propan-2-ol, Bis-(2-hydroxy-5-aminophenyl)methan, 1,3-Bis-(2,5-diaminophenoxy)-propan-2-ol, N,N'-Bis-(4-aminophenyl)-1,4-diazacycloheptan, 1,10-Bis-(2,5-diaminophenyl)-1,4,7,10-tetraoxadecan oder eines ihrer physiologisch verträglichen Salze. Weiterhin kann es erfindungsgemäß bevorzugt sein, als Entwicklerkomponente ein p-Aminophenolderivat oder eines seiner physiologisch verträglichen Salze einzusetzen. Bevorzugte p-Aminophenole sind insbesondere p-Aminophenol, N-Methyl-p-aminophenol, 4-Amino-3-methyl-phenol, 4-Amino-3-fluorphenol, 2-Hydroxymethylamino-4-aminophenol, 4-Amino-3-hydroxymethylphenol, 4-Amino-2-(2-hydroxyethoxy)-phenol, 4-Amino-2-methylphenol, 4-Amino-2-hydroxymethylphenol, 4-Amino-2-methoxymethyl-phenol, 4-Amino-2-aminomethylphenol, 4-Amino-2-(2-hydroxyethyl-aminomethyl)-phenol, 4-Amino-2-(1,2-dihydroxyethyl)-phenol, 4-Amino-2-fluorphenol, 4-Amino-2-chlorphenol, 4-Amino-2,6-dichlorphenol, 4-Amino-2-(diethylaminomethyl)phenol sowie ihre physiologisch verträglichen Salze. Ganz besonders bevorzugte Verbindungen sind p-Aminophenol, 4-Amino-3-methylphenol, 4-Amino-2-aminomethylphenol, 4-Amino-2-(1,2-dihydroxyethyl)phenol und 4-Amino-2-(diethylaminomethyl)phenol. Ferner kann die Entwicklerkomponente ausgewählt sein aus o-Aminophenol und seinen Derivaten, wie 2-Amino-4-methylphenol, 2-Amino-5-methylphenol oder 2-Amino-4-chlorphenol. Weiterhin kann die Entwicklerkomponente ausgewählt sein aus heterocyclischen Entwicklerkomponenten, wie Pyrimidinderivaten, Pyrazolderivaten, Pyrazolopyrimidin-und Pyrazolopyrazol-Derivaten bzw. ihren physiologisch verträglichen Salzen. Bevorzugte Pyrimidin-Derivate sind insbesondere die Verbindungen 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin. Bevorzugte Pyrazol-Derivate sind insbesondere die Verbindungen, die ausgewählt werden unter 4,5-Diamino-1-methylpyrazol, 4,5-Diamino-1-(2-hydroxyethyl)pyrazol, 3,4-Diaminopyrazol, 4,5-Diamino-1-(4'-chlorbenzyl)pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-t-butyl-1-methylpyrazol, 4,5-Diamino-1-t-butyl-3-methylpyrazol, 4,5-Diamino-1-(2-hydroxyethyl)-3-methylpyrazol, 4,5-Diamino-1-ethyl-3-methylpyrazol, 4,5-Diamino-1-ethyl-3-(4-methoxyphenyl)pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropyl-pyrazol, 4-Amino-5-(2-aminoethyl)amino-1,3-dimethylpyrazol, sowie deren physiologisch verträglichen Salze, insbesondere jedoch 4,5-Diamino-1-(2-hydroxyethyl)pyrazol. Bevorzugte Pyrazolopyrimidine sind die Verbindungen, die ausgewählt werden unter Pyrazolo[1,5-a]pyrimidin-3,7-diamin, 2,5-Dimethyl-pyrazolo[1,5-a]pyrimidin-3,7-diamin, Pyrazolo[1,5-a]pyrimidin-3,5-diamin, 2,7-Dimethyl-pyrazolo[1,5-a]pyrimidin-3,5-diamin, 3-Aminopyrazolo[1,5-a]pyrimidin-7-ol, 3-Aminopyrazolo[1,5-a]pyrimidin-5-ol, 2-(3-Aminopyrazolo[1,5-a]pyrimidin-7-ylamino)ethanol, 2-(7-Aminopyrazolo[1,5-a]pyrimidin-3-ylamino)ethanol, 2-[(3-Aminopyrazolo[1,5-a]pyrimidin-7-yl)-(2-hydroxy-ethyl)-amino]ethanol, 2-[(7-Aminopyrazolo[1,5-a]pyrimidin-3-yl)-(2-hydroxyethyl)amino]ethanol, 5,6-Dimethylpyrazolo[1,5-a]pyrimidin-3,7-diamin,2,6-Dimethylpyrazolo[1,5-a]pyrimidin-3,7-diamin, 3-Amino-7-dimethylamino-2,5-dimethylpyrazolo[1,5-a]pyrimidin sowie ihre physiologisch verträglichen Salze und ihre tautomeren Formen, wenn ein tautomeres Gleichgewicht vorhanden ist. Bevorzugtes Pyrazolopyrazol-Derivat ist 2,3-Diamino-6,7-dihydro-1H,5H-pyrazolo[1 ,2-a]pyrazol-1-on.

Besonders bevorzugte Entwicklerkomponenten werden ausgewählt aus mindestens einer Verbindung aus der Gruppe p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1H-imidazol-1-yl)propyl]amin, N,N'-Bis-(2-hydroxyethyl)-N,N'-bis-(4-aminophenyl)-1,3-diamino-propan-2-ol, Bis-(2-hydroxy-5-aminophenyl)-methan, 1,3-Bis-(2,5-diaminophenoxy)propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-aminomethylphenol, 4-Amino-2-(1,2-dihydroxyethyl)phenol, 4-Amino-2-(diethylaminomethyl)-phenol, 4,5-Diamino-1-(2-hydroxyethyl)pyrazol, 2,4,5,6-Tetraaminopyrimidin, 4-Hydroxy-2,5,6-triaminopyrimidin, 2-Hydroxy-4,5,6-triaminopyrimidin, 2,3-Diamino-6,7-dihydro-1H,5H-pyrazolo[1,2-a]pyrazol-1-on sowie deren physiologisch verträglichen Salzen. Ganz besonders bevorzugte Entwicklerkomponenten sind p-Toluylendiamin, 2-(2-Hydroxyethyl)-p-phenylendiamin, 2-Methoxymethyl-p-phenylendiamin, N-(4-Amino-3-methylphenyl)-N-[3-(1H-imidazol-1-yl)propyl]amin, und/oder 4,5-Diamino-1-(2-hydroxyethyl)-pyrazol sowie deren physiologisch verträglichen Salze.

Es hat sich gezeigt, daß sich bestimmte Oxidationsfarbstoffvorprodukte vom Entwicklertyp in bestimmten Mengen besonders gut eignen, um im Vorbehandlungsmittel (A) eingesetzt zu werden und bei dessen Anwendungen zu besonders lebendigen, wasch-, reib-, schweiß- und UV-echten multiotonalen Färbungen zu führen.

Besonders bevorzugte erfindungsgemäße Verfahren sind dadurch gekennzeichnet, daß das Mittel (A) als Oxidationsfarbstoffvorprodukt vom Entwicklertyp (a1) ein oder mehrere Verbindungen aus der Gruppe p-Phenylendiamin, p-Toluylendiamin, 2-(2-Hydroxyethyl)-p-phenylendiamin, 2-(1,2-Dihydroxy-ethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1H-imidazol-1-yl)propyl]amin und/oder deren physiologisch verträgliche Salze in einer Gesamtmenge von 0,1 bis 3,5 Gew.-%, bevorzugt von 0,3 bis 2,8 Gew.-%, weiter bevorzugt von 0,4 bis 2,1 Gew.-% und besonders bevorzugt von 0,5 bis 1,6 Gew.-% - bezogen auf das Gesamtgewicht des Mittels (A) - enthält.

Weitere besonders bevorzugte erfindungsgemäße Verfahren sind dadurch gekennzeichnet, daß das Mittel (A) als Oxidationsfarbstoffvorprodukt vom Entwicklertyp (a1) ein oder mehrere Verbindungen aus der Gruppe Bis-(2-hydroxy-5-aminophenyl)methan, 1,3-Bis-(2,5-diaminophenoxy)propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-und Amino-3-methylphenol und/oder deren physiologisch verträgliche Salzen in einer Gesamtmenge von 0,1 bis 3,5 Gew.-%, bevorzugt von 0,3 bis 2,8 Gew.-%, weiter bevorzugt von 0,4 bis 2,1 Gew.-% und besonders bevorzugt von 0,5 bis 1,6 Gew.-% - bezogen auf das Gesamtgewicht des Mittels (A) - enthält.

Weitere besonders bevorzugte erfindungsgemäße Verfahren sind dadurch gekennzeichnet, daß das Mittel (A) als Oxidationsfarbstoffvorprodukt vom Entwicklertyp (a1) ein oder mehrere Verbindungen aus der Gruppe 4,5-Diamino-1-(2-hydroxyethyl)pyrazol, 2,4,5,6-Tetraaminopyrimidin, 4-Hydroxy-2,5,6-triaminopyrimidin, 2-Hydroxy-4,5,6-triaminopyrimidin, 2,3-Diamino-6,7-dihydro-1H,5H-pyrazolo[1,2-a]pyrazol-1-on und/oder deren physiologisch verträglichen Salzen in einer Gesamtmenge von 0,1 bis 3,5 Gew.-%, bevorzugt von 0,3 bis 2,8 Gew.-%, weiter bevorzugt von 0,4 bis 2,1 Gew.-% und besonders bevorzugt von 0,5 bis 1,6 Gew.-% - bezogen auf das Gesamtgewicht des Mittels (A) - enthält.

Weitere besonders bevorzugte erfindungsgemäße Verfahren sind dadurch gekennzeichnet, daß das Mittel (A) als Oxidationsfarbstoffvorprodukte vom Entwicklertyp (a1) mindestens eine der folgenden Kombinationen enthält: p-Toluylendiamin/2-(2-Hydroxyethyl)-p-phenylendiamin; p-Toluylendiamin/2-Methoxymethyl-p-phenylendiamin; p-Toluylendiamin/N,N-Bis-(2-hydroxyethyl)-p-phenylendiamin; p-Toluylendiamin/2-Methoxymethyl-p-phenylendiamin; p-Toluylendiamin/N-(4-Amino-3-methylphenyl)-N-[3-(1H-imidazol-1-yl)propyl]amin; p-Toluylendiamin/Bis-(2-hydroxy-5-aminophenyl)methan; p-Toluylendiamin/4-und Amino-3-methylphenol; p-Toluylendiamin/4,5-Diamino-1-(2-hydroxyethyl)pyrazol; p-Toluylendiamin/2,4,5,6-Tetraaminopyrimidin; 2-(2-Hydroxyethyl)-p-phenylendiamin /2-Methoxymethyl-p-phenylendiamin; 2-(2-Hydroxyethyl)-p-phenylendiamin /N,N-Bis-(2-hydroxyethyl)-p-phenylendiamin; 2-(2-Hydroxyethyl)-p-phenylendiamin /2-Methoxymethyl-p-phenylendiamin; 2-(2-Hydroxyethyl)-p-phenylendiamin /N-(4-Amino-3-methylphenyl)-N-[3-(1H-imidazol-1-yl)propyl]amin; 2-(2-Hydroxyethyl)-p-phenylendiamin /Bis-(2-hydroxy-5-aminophenyl)methan; 2-(2-Hydroxyethyl)-p-phenylendiamin/4-Amino-3-methylphenol; 2-(2-Hydroxyethyl)-p-phenylendiamin /4,5-Diamino-1-(2-hydroxyethyl)pyrazol; 2-(2-Hydroxyethyl)-p-phenylendiamin /2,4,5,6-Tetraaminopyrimidin; 2-Methoxymethyl-p-phenylendiamin/2-(2-Hydroxyethyl)-p-phenylendiamin; 2-Methoxymethyl-p-phenylendiamin /2-Methoxymethyl-p-phenylendiamin; 2-Methoxymethyl-p-phenylendiamin /N,N-Bis-(2-hydroxyethyl)-p-phenylendiamin; 2-Methoxymethyl-p-phenylendiamin /2-Methoxymethyl-p-phenylendiamin; 2-Methoxymethyl-p-phenylendiamin /N-(4-Amino-3-methylphenyl)-N-[3-(1H-imidazol-1-yl)propyl]amin; 2-Methoxymethyl-p-phenylendiamin /Bis-(2-hydroxy-5-aminophenyl)methan; 2-Methoxymethyl-p-phenylendiamin/4-Amino-3-methylphenol; 2-Methoxymethyl-p-phenylendiamin /4,5-Diamino-1-(2-hydroxyethyl)pyrazol; 2-Methoxymethyl-p-phenylendiamin /2,4,5,6-Tetraaminopyrimidin und/oder 4- und Amino-3-methylphenol/4,5-Diamino-1-(2-hydroxyethyl)pyrazol und/oder deren physiologisch verträgliche Salze.

Erfindungsgemäß enthält das Mittel (A) weiterhin ein oder mehrere Oxidationsfarbstoffvorprodukte vom Kupplertyp (a2).

Kupplerkomponenten bilden im Rahmen der oxidativen Färbung allein keine signifikante Färbung aus, sondern benötigen stets die Gegenwart von Entwicklerkomponenten. Daher ist es erfindungsgemäß bevorzugt, daß bei Verwendung mindestens einer Kupplerkomponente zusätzlich mindestens eine Entwicklerkomponente zum Einsatz kommt. Kupplerkomponenten im Sinne der Erfindung erlauben mindestens eine Substitution eines chemischen Restes des Kupplers durch die oxidierte Form der Entwicklerkomponente. Dabei bildet sich eine kovalente Bindung zwischen Kuppler- und Entwicklerkomponente aus.

Erfindungsgemäße Kupplerkomponenten werden bevorzugt als mindestens eine Verbindung aus einer der folgenden Klassen ausgewählt: m-Aminophenol, o-Aminophenol, m-Diaminobenzol, o-Diaminobenzol und/oder deren Derivate; Naphthalinderivate mit mindestens einer Hydroxygruppe; Dibeziehungsweise Trihydroxybenzol; Pyridinderivate; Pyrimidinderivate; bestimmte Indol-Derivate und Indolin-Derivate; Pyrazolonderivate (beispielsweise 1-Phenyl-3-methylpyrazol-5-on); Morpholinderivate (beispielsweise 6-Hydroxybenzomorpholin oder 6-Aminobenzomorpholin); Chinoxalinderivate (beispielsweise 6-Methyl-1,2,3,4-tetrahydrochinoxalin), sowie Gemische aus zwei oder mehreren Verbindungen aus einer oder mehreren dieser Klassen.

Bevorzugte m-Aminophenol-Kupplerkomponenten werden ausgewählt aus mindestens einer Verbindung aus der Gruppe 3-Aminophenol, 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)amino-2-methylphenol, 3-Diethylaminophenol, N-Cyclopentyl-3-aminophenol, 1,3-Dihydroxy-5-(methylamino)benzol, 3-Ethylamino-4-methylphenol, 2,4-Dichlor-3-aminophenol und deren physiologisch verträglichen Salzen. Bevorzugte m-Diaminobenzol-Kupplerkomponenten werden ausgewählt aus mindestens einer Verbindung aus der Gruppe m-Phenylendiamin, 2-(2,4-Diaminophenoxy)ethanol, 1,3-Bis(2,4-diaminophenoxy)propan, 1-Methoxy-2-amino-4-(2'-hydroxyethylamino)benzol, 1,3-Bis(2,4-diaminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2'-hydroxyethyl)aminobenzol und deren physiologisch verträglichen Salzen. Bevorzugte o-Diaminobenzol-Kupplerkomponenten werden ausgewählt aus mindestens einer Verbindung aus der Gruppe 3,4-Diaminobenzoesäure und 2,3-Diamino-1-methylbenzol und deren physiologisch verträglichen Salzen. Bevorzugte Naphthalinderivate mit mindestens einer Hydroxygruppe werden ausgewählt aus mindestens einer Verbindung der Gruppe 1-Naphthol, 2-Methyl-1-naphthol, 2-Hydroxymethyl-1-naphthol, 2-Hydroxyethyl-1-naphthol, 1,3-Dihydroxynaphthalin, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin. Bevorzugte Di- beziehungsweise Trihydroxybenzole und deren Derivate werden ausgewählt aus mindestens einer Verbindung der Gruppe Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol. Bevorzugte Pyridinderivate werden ausgewählt aus mindestens einer Verbindung der Gruppe 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin, 3,5-Diamino-2,6-dimethoxypyridin, 3,4-Diaminopyridin, 2-(2-Methoxyethyl)amino-3-amino-6-methoxypyridin, 2-(4'-Methoxyphenyl)amino-3-aminopyridin, und deren physiologisch verträglichen Salzen. Bevorzugte Pyrimidinderivate werden ausgewählt aus mindestens einer Verbindung der Gruppe 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin und deren physiologisch verträglichen Salzen. Bevorzugte Indolderivate werden ausgewählt aus mindestens einer Verbindung der Gruppe 4-Hydroxyindol, 6-Hydroxyindol und 7-Hydroxyindol und deren physiologisch verträglichen Salzen. Bevorzugte Indolinderivate werden ausgewählt aus mindestens einer Verbindung der Gruppe 4-Hydroxyindolin, 6-Hydroxyindolin und 7-Hydroxyindolin und deren physiologisch verträglichen Salzen.

Erfindungsgemäß besonders bevorzugte Kupplerkomponenten werden ausgewählt unter 3-Aminophenol, 5-Amino-2-methylphenol, 3-Amino-2-chlor-6-methylphenol, 2-Hydroxy-4-aminophenoxy-ethanol, 5-Amino-4-chlor-2-methylphenol, 5-(2-Hydroxyethyl)-amino-2-methylphenol, 2,4-Dichlor-3-aminophenol, 2-Aminophenol, 3-Phenylendiamin, 2-(2,4-Diaminophenoxy)ethanol, 1,3-Bis(2,4-diaminophenoxy)propan, 1-Methoxy-2-amino-4-(2-hydroxyethylamino)benzol, 1,3-Bis(2,4-diaminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}-amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2-hydroxyethyl)aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Dihydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin, 7-Hydroxyindolin oder Gemischen dieser Verbindungen oder deren physiologisch verträglichen Salzen. Ganz besonders bevorzugt sind Resorcin, 2-Methylresorcin, 5-Amino-2-methylphenol, 3-Aminophenol, 2-(2,4-Diaminophenoxy)ethanol, 1,3-Bis(2,4-diaminophenoxy)propan, 1-Methoxy-2-amino-4-(2'-hydroxyethylamino)benzol, 2-Amino-3-hydroxypyridin und 1-Naphthol sowie eines deren physiologisch verträglichen Salze.

Die Kupplerkomponenten werden bevorzugt in einer Menge von 0,0001 bis 0,5 Gew.-%, vorzugsweise 0,001 bis 0,2 Gew.-%, jeweils bezogen auf das Mittel (A), verwendet.

Es hat sich gezeigt, daß sich bestimmte Oxidationsfarbstoffvorprodukte vom Kupplertyp in bestimmten Mengen besonders gut eignen, um im Vorbehandlungsmittel (A) eingesetzt zu werden und bei dessen Anwendungen zu besonders lebendigen, wasch-, reib-, schweiß- und UV-echten multitonalen Färbungen zu führen.

Erfindungsgemäß bevorzugte Verfahren sind daher dadurch gekennzeichnet, daß das Mittel (A) als Oxidationsfarbstoffvorpdodukt vom Kupplertyp (a2) ein oder mehrere Verbindungen aus der Gruppe 3-Aminophenol, 5-Amino-2-methylphenol, 3-Amino-2-chlor-6-methylphenol, 2-Hydroxy-4-aminophenoxy-ethanol, 5-Amino-4-chlor-2-methylphenol, 5-(2-Hydroxyethyl)-amino-2-methylphenol, 2,4-Dichlor-3-aminophenol, 2-Aminophenol und/oder deren physiologisch verträgliche Salze in einer Gesamtmenge von 0,1 bis 3,5 Gew.-%, bevorzugt von 0,3 bis 2,8 Gew.-%, weiter bevorzugt von 0,4 bis 2,1 Gew.-% und besonders bevorzugt von 0,5 bis 1,6 Gew.-% - bezogen auf das Gesamtgewicht des Mittels (A) - enthält.

Weitere erfindungsgemäß bevorzugte Verfahren sind dadurch gekennzeichnet, daß das Mittel (A) als Oxidationsfarbstoffvorpdodukt vom Kupplertyp (a2) ein oder mehrere Verbindungen aus der Gruppe 3-Phenylendiamin, 2-(2,4-Diaminophenoxy)ethanol, 1,3-Bis(2,4-diaminophenoxy)propan, 1-Methoxy-2-amino-4-(2-hydroxyethylamino)benzol, 1,3-Bis(2,4-diaminophenyl)propan, 2,6-Bis(2'-hydroxyethylamino)-1-methylbenzol und/oder deren physiologisch verträgliche Salze in einer Gesamtmenge von 0,1 bis 3,5 Gew.-%, bevorzugt von 0,3 bis 2,8 Gew.-%, weiter bevorzugt von 0,4 bis 2,1 Gew.-% und besonders bevorzugt von 0,5 bis 1,6 Gew.-% - bezogen auf das Gesamtgewicht des Mittels (A) - enthält.

Weitere erfindungsgemäß bevorzugte Verfahren sind dadurch gekennzeichnet, daß das Mittel (A) als Oxidationsfarbstoffvorpdodukt vom Kupplertyp (a2) ein oder mehrere Verbindungen aus der Gruppe Resorcin, 2-Methylresorcin und/oder 4-Chlorresorcin in einer Gesamtmenge von 0,1 bis 3,5 Gew.-%, bevorzugt von 0,3 bis 2,8 Gew.-%, weiter bevorzugt von 0,4 bis 2,1 Gew.-% und besonders bevorzugt von 0,5 bis 1,6 Gew.-% - bezogen auf das Gesamtgewicht des Mittels (A) - enthält.

Weitere erfindungsgemäß bevorzugte Verfahren sind dadurch gekennzeichnet, daß das Mittel (A) als Oxidationsfarbstoffvorpdodukt vom Kupplertyp (a2) ein oder mehrere Verbindungen aus der Gruppe 2-Amino-3-hydroxypyridin, 3-Amino-2-methylamino-6-methoxypyridin, 2,6-Dihydroxy-3,4-dimethylpyridin, 3,5-Diamino-2,6-dimethoxypyridin, 1-Phenyl-3-methylpyrazol-5-on und/oder deren physiologisch verträgliche Salze in einer Gesamtmenge von 0,1 bis 3,5 Gew.-%, bevorzugt von 0,3 bis 2,8 Gew.-%, weiter bevorzugt von 0,4 bis 2,1 Gew.-% und besonders bevorzugt von 0,5 bis 1,6 Gew.-% - bezogen auf das Gesamtgewicht des Mittels (A) - enthält.

Weitere erfindungsgemäß bevorzugte Verfahren sind dadurch gekennzeichnet, daß das Mittel (A) als Oxidationsfarbstoffvorpdodukt vom Kupplertyp (a2) mindestens eine der folgenden Kombinationen enthält: Resorcin/3-Aminophenol; 2-Methylresorcin/3-Aminophenol; 4-Chlorresorcin/3-Aminophenol; Resorcin/5-Amino-2-methylphenol; 2-Methylresorcin/5-Amino-2-methylphenol; 4-Chlorresorcin/5-Amino-2-methylphenol; Resorcin/2-Hydroxy-4-aminophenoxyethanol; 2-Methylresorcin/2-Hydroxy-4-aminophenoxyethanol; 4-Chlorresorcin/2-Hydroxy-4-aminophenoxyethanol; Resorcin/2-Amino-3-hydroxypyridin; 2-Methylresorcin/2-Amino-3-hydroxypyridine; 4-Chlorresorcin/2-Amino-3-hydroxypyridine; Resorcin/3-Amino-2-methylamino-6-methoxypyridin; 2-Methylresorcin/3-Amino-2-methylamino-6-methoxypyridin; 4-Chlorresorcin/3-Amino-2-methylamino-6-methoxypyridin; Resorcin/2,6-Dihydroxy-3,4-dimethylpyridin; 2-Methylresorcin/2,6-Dihydroxy-3,4-dimethylpyridin und/oder 4-Chlorresorcin/2,6-Dihydroxy-3,4-dimethylpyridin und/oder deren physiologisch verträglichen Salze.

Das Vorbehandlungsmittel (A) besitzt einen pH-Wert von 8,0 bis 12,0 (a3). Bevorzugte erfindungsgemäße Verfahren sind dadurch gekennzeichnet, daß das Mittel (A), einen pH-Wert (a3) von 8,5 bis 11,5, bevorzugt von 8,8 bis 11,0, weiter bevorzugt von 9,0 bis 10,8 und besonders bevorzugt von 9,2 bis 10,5 besitzt.

Zusätzlich zu dem bzw. den Oxidationsfarbstoffvorprodukt(en) vom Entwicklertyp und dem bzw. den Oxidationsfarbstoffvorprodukt(en) vom Kupplertyp kann das im erfindungsgemäßen Verfahren eingesetzte Mittel (A) weitere Inhaltsstoffe enthalten. Hier haben sich insbesondere Alkalisierungsmittel als besonders geeignet erwiesen.

Erfindungsgemäß einsetzbare, organische Alkalisierungsmittel werden bevorzugt ausgewählt aus Alkanolaminen aus primären, sekundären oder tertiären Aminen mit einem C₂-C₆-Alkylgrundkörper, der mindestens eine Hydroxylgruppe trägt. Erfindungsgemäß ganz besonders bevorzugte Alkanolamine werden ausgewählt aus der Gruppe 2-Aminoethan-1-ol (Monoethanolamin), 2-Amino-2-methylpropan-1-ol und 2-Amino-2-methyl-propan-1,3-diol. Ein insbesondere bevorzugtes Alkanolamin ist Monoethanolamin. Geeignete basische Aminsäuren sind Lysin, Arginin und Ornithin. Das erfindungsgemäße, anorganische Alkalisierungsmittel sind bevorzugt ausgewählt aus der Gruppe Natriumhydroxid, Kaliumhydroxid, Calciumhydroxid, Bariumhydroxid, Natriumphosphat, Kaliumphosphat, Natriumsilicat, Kaliumsilicat, Natriumcarbonat und Kaliumcarbonat.

Erfindungsgemäß besonders bevorzugte Verfahren sind dadurch gekennzeichnet, daß das Mittel (A), ein oder mehrere Alkalisierungsmittel aus der Gruppe Natriumhydroxid, Kaliumhydroxid, Ammoniak, Monoethanolamin und 2-Amino-2-methylpropanol in einer Gesamtmenge von 0,3 bis 4,5 Gew.-%, bevorzugt 0,5 bis 3,5 Gew.-%, weiter bevorzugt von 0,7 bis 2,5 Gew.-% und besonders bevorzugt von 0,9 bis 1,5 Gew.-% - bezogen auf das Gesamtgewicht des Mittels (A) - enthält.

Um das Vorpenetrationsmittel (A) sauber und lokal begrenzt applizieren zu können, hat sich eine höhere Viskosität des Mittels als vorteilhaft erwiesen. Dazu ist vorteilhaft, wenn das Mittel nicht als Paste, zähe Creme oder eingedicktes Gel vorliegt, sondern über eine ausreichende Fließfähigkeit verfügt. Weiterhin muß das anwendungsbereite Mittel zwar über rheologische Eigenschaften verfügen, die ein Auftragen auf die zu färbenden Fasern erlauben, aber gleichzeitig ein Verlaufen oder Ausfliessen des Mittels vom Wirkort während Anwendungsdauer verhindern. Bevorzugt besitzen die Mittel (A) daher eine Viskosität von 5 bis 100 Pa·s, bevorzugt von 10 bis 50 Pa·s, insbesondere von 10 bis 20 Pa·s und insbesondere bevorzugt von 10 bis 16 Pa·s (Brookfield, 22°C, Spindel #5, 4 rpm). Hierzu enthalten bevorzugte Mittel (A) mindestens ein Verdickungsmittel und/oder mindestens einen Gelbildner. Entsprechende erfindungsgemäße Verfahren, bei denen das Mittel (A), zusätzlich mindestens ein Verdickungsmittel und/oder mindestens einen Gelbildner enthält, sind erfindungsgemäß bevorzugt.

Eine weitere ganz besonders bevorzugte Ausführungsform ist dadurch gekennzeichnet, dass das Vorpenetrationsmittel (A) eine Viskosität von 5000 bis 12 000 mPas, bevorzugt von 5500 bis 11 000 mPas, weiter bevorzugt von 6000 bis 10 000 mPas und ganz besodners bevorzugt von 6500 bis 9500 mPas (Brookfield, 22°C, Spindel #5, 4 rpm) besitzt.

Besonders bevorzugte Vorpenetrationsmittel (A) sind in einer weiteren Ausführungsform daher dadurch gekennzeichnet, daß sie mindestens ein anionisches, polymeres Verdickungsmittel enthalten. Bevorzugte anionische polymere Verdickungsmittel sind ausgewählt aus vernetzten oder unvernetzten Copolymeren, die mindestens zwei unterschiedliche Monomere aus der Gruppe der Acrylsäure, Methacrylsäure, den C₁-C₆-Alkylestern der Acrylsäure und/oder den C₁-C₆-Alkylestern der Methacrylsäure enthalten.

Besonders bevorzugte anionische Copolymere sind Copolymere aus Acrylsäure, Methacrylsäure oder deren C₁-C₆-Alkylestern, die unter der INCI-Bezeichnung Acrylates Copolymer vertrieben werden. Insbesondere bevorzugt ist die Kombination aus Methacrylsäure und Ethylacrylat sowie gegebenenfalls vernetzenden, multifunktionalen Monomeren. Ein bevorzugtes Handelsprodukt dafür ist beispielsweise Aculyn^{®} 33 bzw. 33A, das von der Firma Rohm & Haas angeboten wird.

Als weitere bevorzugte Verdickungsmittel kann das Vorpenetrationsmittel (A) ein oder mehrere anionische, polymere Verdicker aus der Gruppe der Xanthane, Alginate, Carboxyalkylcellulosen und Hyaluronsäuren enthalten.

Xanthan ist ein anionisches Polysaccharid, welches unter anderem aus den Strukturbestandteilen D-Glucose, D-Mannose, D-Glucuronsäure, Acetat und Pyruvat aufgebaut ist und das auch unter dem INCI Namen Xanthan Gum bekannt ist.

Als Alginate (INCI-Name Algin) werden die Salze der Alginsäure bezeichnet. Alginate sind saure, Carboxy-Gruppen enthaltende Polysaccharide, bestehend aus D-Mannuronsäure und D-Guluronsäure in unterschiedlichen Verhältnissen, welche mit 1-4-glycosidischen Bindungen verknüpft sind. Erfindungsgemäß sind sowohl die Alkali- als auch die Erdalkalisalze der Alignsäuren. Besonders vorteilhaft hat sich der Einsatz von Alginsäure, Natriumalginat, Kaliumalginat, Ammoniumalginat und/oder Calciumalginat in den erfindungsgemäßen Mitteln herausgestellt.

Carboxyalkylcellulosen sind Celluloseether, bei denen die Wasserstoffatome der Hydroxygruppen der Cellulose teilweise oder vollständig durch Carboxyalkylgruppen substituiert sind. Eine bevorzugte Carboxyalkylcellulose ist die Carboxymethylcellulose, die vorzugsweise in Form ihres Natriumsalzes (Natrium-Carboxymethylcellulose) als anionisches Polymer Einsatz finden kann.

Grundbausteine der Hyaluronsäure (INCI Namen Hyaluronic acid, Sodium Hyaluronat) ist ein aus D-Glucuronsäure und N-Acetylglucsoamin in 1-3-glykosidischer Bindung aufgebautes Aminodisaccharid, das mit der nächsten Einheit ⇓-1-4-glykosidisch verbunden ist. Natrium- und Kaliumsalze der Hyaluronsäure haben sich im Rahmen der zu dieser Erfindung führenden Arbeiten als besonders geeignet zur Erzeugung von intensiv färbenden und im Hinblick auf ihre Viskosität optimierten Färbeformulierungen erwiesen.

Eine weitere ganz besonders bevorzugte Ausführungsform ist dadurch gekennzeichnet, dass das Vorpenetrationsmittel (A) ein oder mehrere anionische, polymere Verdickungsmittel aus der Gruppe der Copolymere aus Acrylsäure und C₁-C₆-Alkylestern, der Copolymere aus Methacrylsäure und C₁-C₆-Alkylestern, Xanthan und Caroboxymethylcellulose enthält.

Die anionischen polymeren Verdickungsmittel können bevorzugt in einer Gesamtmenge von 0,1 bis 15 Gew.-%, mehr bevorzugt von 1 bis 10 Gew.-% und insbesondere von 1,5 bis 7,5 Gew.-% eingesetzt werden, wobei sich die Mengen auf das Gesamtgewicht des Vorpenetrationsmittel (A) beziehen.

Um das natürliche und multitonale Färbeergebnis am Ende des erfindungsgemäßen Verfahrens besonders deutlich und überraschend hervortreten zu lasen, ist das Vorbehandlungsmittel (A) für sich alleine nicht in der Lage, als separates Bleich-, Aufhell- oder Färbemittel eingesetzt zu werden. Hierzu ist es von besonderem Vorteil, wenn die applikationsbereiten Mittel (A) frei sind von Oxidationsmitteln, insbesondere frei sind von Wasserstoffperoxid.

Dabei bedeutet "frei von", daß keine intentionell zugesetzten Verbindungen aus den genannten Gruppen in den Mitteln enthalten sind. Gleichwohl können Spuren dieser Verbindungen als Verunreinigung oder Begleitstoff über andere Rohstoffe in die Mittel eingebracht werden. "Frei von" bedeutet daher konkreter, daß die applikationsbereiten Mittel (A) - bezogen auf ihr Gewicht - weniger als 1 Gew.-%, vorzugsweise weniger als 0,5 Gew.-%, weiter bevorzugt weniger als 0,25 Gew.-%, noch weiter bevorzugt weniger als 0,1 Gew.-% und insbesondere weniger als 0,01 Gew.-% Verbindungen aus den genannten Gruppen enthalten.

Erfindungsgemäße Verfahren sind dadurch gekennzeichnet, daß das in Schritt A) eingesetzte Mittel (A) - bezogen auf sein Gewicht - weniger als 0,01 Gew.-% Wasserstoffperoxid enthält.

Erfindungsgemäß weiter bevorzugte Verfahren sind dadurch gekennzeichnet, daß das in Schritt A) eingesetzte Mittel (A) - bezogen auf sein Gewicht - weniger als 1 Gew.-%, vorzugsweise weniger als 0,5 Gew.-%, weiter bevorzugt weniger als 0,25 Gew.-%, noch weiter bevorzugt weniger als 0,1 Gew.-% und insbesondere weniger als 0,01 Gew.-% Peroxoverbindungen enthält.

Das Vorbehandlungsmittel (A) kann weitere übliche Inhaltstoffe enthalten, diese werden weiter unten ausführlich beschrieben.

In Schritt C) des erfindungsgemäßen Verfahrens wird ein kosmetisches Mittel (B) auf die keratinischen Fasern appliziert, die noch mit dem Mittel (A) beaufschlagt sind. Dieses Mittel (B) enthält ein oder mehrere Oxidationsfarbstoffvorprodukte (b1) und ein oder mehrere Oxidationsmittel (b2) und weist einen pH-Wert von 8,0 bis 12,0 auf.

Bevorzugte Mittel (B) enthalten mindestens ein Oxidationsfarbstoffvorprodukt vom Entwicklertyp. Entsprechende erfindungsgemäße Verfahren, bei denen das Mittel (B) als Oxidationsfarbstoffvorprodukt (b1) ein oder mehrere Oxidationsfarbstoffvorprodukte vom Entwicklertyp enthält, sind erfindungsgemäß bevorzugt.

Geeignete und bevorzugte Oxidationsfarbstoffvorprodukte vom Entwicklertyp wurden bereits weiter oben detailliert beschrieben. Die entsprechenden Verbindungen sind auch im Mittel (B) mit Vorzug einsetzbar. Es hat sich gezeigt, daß sich bestimmte Oxidationsfarbstoffvorprodukte vom Entwicklertyp in bestimmten Mengen besonders gut eignen, um im Mittel (B) eingesetzt zu werden und bei dessen Anwendungen zu besonders lebendigen, wasch-, reib-, schweiß- und UV-echten multitonalen Färbungen zu führen.

Besonders bevorzugte erfindungsgemäße Verfahren sind dadurch gekennzeichnet, daß das Mittel (B) als Oxidationsfarbstoffvorprodukt vom Entwicklertyp ein oder mehrere Verbindungen aus der Gruppe p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1H-imidazol-1-yl)propyl]amin und/oder deren physiologisch verträgliche Salze in einer Gesamtmenge von 0,1 bis 3,5 Gew.-%, bevorzugt von 0,3 bis 2,8 Gew.-%, weiter bevorzugt von 0,4 bis 2,1 Gew.-% und besonders bevorzugt von 0,5 bis 1,6 Gew.-% - bezogen auf das Gesamtgewicht des Mittels (B) - enthält.

Weitere besonders bevorzugte erfindungsgemäße Verfahren sind dadurch gekennzeichnet, daß das Mittel (B) als Oxidationsfarbstoffvorprodukt vom Entwicklertyp ein oder mehrere Verbindungen aus der Gruppe Bis-(2-hydroxy-5-aminophenyl)methan, 1,3-Bis-(2,5-diaminophenoxy)propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-und Amino-3-methylphenol und/oder deren physiologisch verträgliche Salzen in einer Gesamtmenge von 0,1 bis 3,5 Gew.-%, bevorzugt von 0,3 bis 2,8 Gew.-%, weiter bevorzugt von 0,4 bis 2,1 Gew.-% und besonders bevorzugt von 0,5 bis 1,6 Gew.-% - bezogen auf das Gesamtgewicht des Mittels (B) - enthält.

Weitere besonders bevorzugte erfindungsgemäße Verfahren sind dadurch gekennzeichnet, daß das Mittel (B) als Oxidationsfarbstoffvorprodukt vom Entwicklertyp ein oder mehrere Verbindungen aus der Gruppe 4,5-Diamino-1-(2-hydroxyethyl)pyrazol, 2,4,5,6-Tetraaminopyrimidin, 4-Hydroxy-2,5,6-triaminopyrimidin, 2-Hydroxy-4,5,6-triaminopyrimidin, 2,3-Diamino-6,7-dihydro-1H,5H-pyrazolo[1,2-a]pyrazol-1-on und/oder deren physiologisch verträglichen Salzen in einer Gesamtmenge von 0,1 bis 3,5 Gew.-%, bevorzugt von 0,3 bis 2,8 Gew.-%, weiter bevorzugt von 0,4 bis 2,1 Gew.-% und besonders bevorzugt von 0,5 bis 1,6 Gew.-% - bezogen auf das Gesamtgewicht des Mittels (B) - enthält.

Weitere besonders bevorzugte erfindungsgemäße Verfahren sind dadurch gekennzeichnet, daß das Mittel (B) als Oxidationsfarbstoffvorprodukte vom Entwicklertyp mindestens eine der folgenden Kombinationen enthält: p-Toluylendiamin/2-(2-Hydroxyethyl)-p-phenylendiamin; p-Toluylendiamin/2-Methoxymethyl-p-phenylendiamin; p-Toluylendiamin/N,N-Bis-(2-hydroxyethyl)-p-phenylendiamin; p-Toluylendiamin/2-Methoxymethyl-p-phenylendiamin; p-Toluylendiamin/N-(4-Amino-3-methylphenyl)-N-[3-(1H-imidazol-1-yl)propyl]amin; p-Toluylendiamin/Bis-(2-hydroxy-5-aminophenyl)methan; p-Toluylendiamin/4-und Amino-3-methylphenol; p-Toluylendiamin/4,5-Diamino-1-(2-hydroxyethyl)pyrazol; p-Toluylendiamin/2,4,5,6-Tetraaminopyrimidin; 2-(2-Hydroxyethyl)-p-phenylendiamin /2-Methoxymethyl-p-phenylendiamin; 2-(2-Hydroxyethyl)-p-phenylendiamin/N,N-Bis-(2-hydroxyethyl)-p-phenylendiamin; 2-(2-Hydroxyethyl)-p-phenylendiamin /2-Methoxymethyl-p-phenylendiamin; 2-(2-Hydroxyethyl)-p-phenylendiamin /N-(4-Amino-3-methylphenyl)-N-[3-(1H-imidazol-1-yl)propyl]amin; 2-(2-Hydroxyethyl)-p-phenylendiamin /Bis-(2-hydroxy-5-aminophenyl)methan; 2-(2-Hydroxyethyl)-p-phenylendiamin/4-Amino-3-methylphenol; 2-(2-Hydroxyethyl)-p-phenylendiamin /4,5-Diamino-1-(2-hydroxyethyl)pyrazol; 2-(2-Hydroxyethyl)-p-phenylendiamin /2,4,5,6-Tetraaminopyrimidin; 2-Methoxymethyl-p-phenylendiamin/2-(2-Hydroxyethyl)-p-phenylendiamin; 2-Methoxymethyl-p-phenylendiamin /2-Methoxymethyl-p-phenylendiamin; 2-Methoxymethyl-p-phenylendiamin /N,N-Bis-(2-hydroxyethyl)-p-phenylendiamin; 2-Methoxymethyl-p-phenylendiamin /2-Methoxymethyl-p-phenylendiamin; 2-Methoxymethyl-p-phenylendiamin /N-(4-Amino-3-methylphenyl)-N-[3-(1H-imidazol-1-yl)propyl]amin; 2-Methoxymethyl-p-phenylendiamin /Bis-(2-hydroxy-5-aminophenyl)methan; 2-Methoxymethyl-p-phenylendiamin/4-Amino-3-methylphenol; 2-Methoxymethyl-p-phenylendiamin /4,5-Diamino-1-(2-hydroxyethyl)pyrazol; 2-Methoxymethyl-p-phenylendiamin /2,4,5,6-Tetraaminopyrimidin und/oder 4- und Amino-3-methylphenol/4,5-Diamino-1-(2-hydroxyethyl)pyrazol und/oder deren physiologisch verträgliche Salze.

Je nach Wunsch, eine multitonale Färbung mit stark oder weniger stark variierenden Nuancierungen bereitzustellen, können die in den Mitteln (A) und (B) eingesetzten Oxidationsfarbstoffvorprodukte voneinander abweichen. Für eine sehr natürlich wirkende multitonale Färbung mit weichen Übergängen sind erfindungsgemäße Verfahren bevorzugt, bei denen die Mittel (A) und (B) gleiche Oxidationsfarbstoffvorprodukte vom Entwicklertyp enthalten.

Werden stärkere Kontraste gewünscht, die sich in einem brillanteren multitonalen Erscheinungsbild der Färbung äußern, haben sich erfindungsgemäße Verfahren bewährt, bei denen die Mittel (A) und (B) unterschiedliche Oxidationsfarbstoffvorprodukte vom Entwicklertyp enthalten.

Erfindungsgemäß bevorzugt enthält das Mittel *(B)* weiterhin ein oder mehrere Oxidationsfarbstoffvorprodukte vom Kupplertyp.

Geeignete und bevorzugte Oxidationsfarbstoffvorprodukte vom Kupplertyp wurden bereits weiter oben detailliert beschrieben. Die entsprechenden Verbindungen sind auch im Mittel (B) mit Vorzug einsetzbar. Es hat sich gezeigt, daß sich bestimmte Oxidationsfarbstoffvorprodukte vom Entwicklertyp in bestimmten Mengen besonders gut eignen, um im Mittel (B) eingesetzt zu werden und bei dessen Anwendungen zu besonders lebendigen, wasch-, reib-, schweiß- und UV-echten multitonalen Färbungen zu führen.

Erfindungsgemäß bevorzugte Verfahren sind daher dadurch gekennzeichnet, daß das Mittel (B) als Oxidationsfarbstoffvorpdodukt vom Kupplertyp ein oder mehrere Verbindungen aus der Gruppe 3-Aminophenol, 5-Amino-2-methylphenol, 3-Amino-2-chlor-6-methylphenol, 2-Hydroxy-4-aminophenoxy-ethanol, 5-Amino-4-chlor-2-methylphenol, 5-(2-Hydroxyethyl)-amino-2-methylphenol, 2,4-Dichlor-3-aminophenol, 2-Aminophenol und/oder deren physiologisch verträgliche Salze in einer Gesamtmenge von 0,1 bis 3,5 Gew.-%, bevorzugt von 0,3 bis 2,8 Gew.-%, weiter bevorzugt von 0,4 bis 2,1 Gew.-% und besonders bevorzugt von 0,5 bis 1,6 Gew.-% - bezogen auf das Gesamtgewicht des Mittels (A) - enthält.

Weitere erfindungsgemäß bevorzugte Verfahren sind dadurch gekennzeichnet, daß das Mittel (B) als Oxidationsfarbstoffvorpdodukt vom Kupplertyp ein oder mehrere Verbindungen aus der Gruppe 3-Phenylendiamin, 2-(2,4-Diaminophenoxy)ethanol, 1,3-Bis(2,4-diaminophenoxy)propan, 1-Methoxy-2-amino-4-(2-hydroxyethylamino)benzol, 1,3-Bis(2,4-diaminophenyl)propan, 2,6-Bis(2'-hydroxyethyl-amino)-1-methylbenzol und/oder deren physiologisch verträgliche Salze in einer Gesamtmenge von 0,1 bis 3,5 Gew.-%, bevorzugt von 0,3 bis 2,8 Gew.-%, weiter bevorzugt von 0,4 bis 2,1 Gew.-% und besonders bevorzugt von 0,5 bis 1,6 Gew.-% - bezogen auf das Gesamtgewicht des Mittels (A) - enthält.

Weitere erfindungsgemäß bevorzugte Verfahren sind dadurch gekennzeichnet, daß das Mittel (B) als Oxidationsfarbstoffvorpdodukt vom Kupplertyp ein oder mehrere Verbindungen aus der Gruppe Resorcin, 2-Methylresorcin und/oder 4-Chlorresorcin in einer Gesamtmenge von 0,1 bis 3,5 Gew.-%, bevorzugt von 0,3 bis 2,8 Gew.-%, weiter bevorzugt von 0,4 bis 2,1 Gew.-% und besonders bevorzugt von 0,5 bis 1,6 Gew.-% - bezogen auf das Gesamtgewicht des Mittels (A) - enthält.

Weitere erfindungsgemäß bevorzugte Verfahren sind dadurch gekennzeichnet, daß das Mittel (B) als Oxidationsfarbstoffvorpdodukt vom Kupplertyp ein oder mehrere Verbindungen aus der Gruppe 2-Amino-3-hydroxypyridin, 3-Amino-2-methylamino-6-methoxypyridin, 2,6-Dihydroxy-3,4-dimethylpyridin, 3,5-Diamino-2,6-dimethoxypyridin, 1-Phenyl-3-methylpyrazol-5-on und/oder deren physiologisch verträgliche Salze in einer Gesamtmenge von 0,1 bis 3,5 Gew.-%, bevorzugt von 0,3 bis 2,8 Gew.-%, weiter bevorzugt von 0,4 bis 2,1 Gew.-% und besonders bevorzugt von 0,5 bis 1,6 Gew.-% - bezogen auf das Gesamtgewicht des Mittels (A) - enthält.

Weitere erfindungsgemäß bevorzugte Verfahren sind dadurch gekennzeichnet, daß das Mittel (B) als Oxidationsfarbstoffvorprodukt vom Kupplertyp mindestens eine der folgenden Kombinationen enthält: Resorcin/3-Aminophenol; 2-Methylresorcin/3-Aminophenol; 4-Chlorresorcin/3-Aminophenol; Resorcin/5-Amino-2-methylphenol; 2-Methylresorcin/5-Amino-2-methylphenol; 4-Chlorresorcin/5-Amino-2-methylphenol; Resorcin/2-Hydroxy-4-aminophenoxyethanol; 2-Methylresorcin/2-Hydroxy-4-aminophenoxyethanol; 4-Chlorresorcin/2-Hydroxy-4-aminophenoxyethanol; Resorcin/2-Amino-3-hydroxypyridin; 2-Methylresorcin/2-Amino-3-hydroxypyridine; 4-Chlorresorcin/2-Amino-3-hydroxypyridine; Resorcin/3-Amino-2-methylamino-6-methoxypyridin; 2-Methylresorcin/3-Amino-2-methylamino-6-methoxypyridin; 4-Chlorresorcin/3-Amino-2-methylamino-6-methoxypyridin; Resorcin/2,6-Dihydroxy-3,4-dimethylpyridin; 2-Methylresorcin/2,6-Dihydroxy-3,4-dimethylpyridin und/oder 4-Chlorresorcin/2,6-Dihydroxy-3,4-dimethylpyridin und/oder deren physiologisch verträglichen Salze.

Wie bereits erwähnt, könne je nach Wunsch, eine multitonale Färbung mit stark oder weniger stark variierenden Nuancierungen bereitzustellen, die in den Mitteln (A) und (B) eingesetzten Oxidationsfarbstoffvorprodukte voneinander abweichen. Für eine sehr natürlich wirkende multitonale Färbung mit weichen Übergängen sind erfindungsgemäße Verfahren bevorzugt, bei denen die Mittel (A) und (B) gleiche Oxidationsfarbstoffvorprodukte vom Kupplerertyp enthalten.

Werden stärkere Kontraste gewünscht, die sich in einem brillanteren multitonalen Erscheinungsbild der Färbung äußern, haben sich erfindungsgemäße Verfahren bewährt, bei denen die Mittel (A) und (B) unterschiedliche Oxidationsfarbstoffvorprodukte vom Kupplertyp enthalten.

Insbesondere bevorzugte erfinddungsgemäße Verfahren sind dadurch gekennzeichnet, daß das Mittel (A) und das Mittel (B) die gleichen Oxidationsfarbstoffvorprodukte enthalten.

Vorzugsweise enthält das Mittel (B) - bezogen auf sein Gewicht - mehr Oxidationsfarbstoffvorprodukte als das Mittel (A).

Erfindungsgemäße Verfahren, bei denen das Mengenverhältnis aus der Gesamtmenge aller Oxidationsfarbstoffvorprodukte im Mittel (A) zur Gesamtmenge aller Oxidationsfarbstoffvorprodukte im Mittel (B) bei einem Wert von 1:1 bis 1:10, bevorzugt von 1:2 bis 1:8, weiter bevorzugt von 1:2 bis 1:5 und besonders bevorzugt von 1:2 bis 1:3 liegt, sind besonders bevorzugt.

Die anwendungsbereiten Mittel (B) enthalten zusätzlich ein oder mehrere Oxidationsmittel (b2). Üblicherweise werden oxidative Färbemittel in Form eines aus zwei Komponenten bestehenden "Kits" (Mehrkomponenten-Verpackungseinheit) angeboten, wobei die erste Komponente die Oxidationsfarbstoffvorprodukte und ein Alkalisierungsmittel (beispielsweise Ammoniak) und die zweite Komponenten das Oxidationsmittel enthält. Als Oxidationsmittel werden in der Regel Peroxide wie beispielsweise Wasserstoffperoxid eingesetzt.

Die Oxidationsfarbstoffvorprodukte (Entwickler und Kuppler) selbst sind nicht gefärbt, sondern die Bildung der eigentlichen Farbstoffe erfolgt erst im Verlauf der Anwendung durch den Kontakt der Oxidationsfarbstoffvorprodukte mit dem Oxidationsmittel (vorzugsweise Wasserstoffperoxid). In chemischer Reaktion werden die als Oxidationsfarbstoffvorprodukte eingesetzten Entwickler (wie beispielsweise p-Phenylendiamin-Derviate oder p-Aminophenolderivate) durch Wasserstoffperoxid zunächst oxidativ in eine reaktive Zwischenstufe, auch Chinonimin oder Chinondiimin genannt, überführt, welche dann in einer oxidativen Kupplungsreaktion mit den Kupplern zum jeweiligen Farbstoff reagiert.

Alle vorstehenden Angaben zu Mittel (B) beziehen sich auf die anwendungsbereite Mischung, auch wenn diese erst unmittelbar vor der Anwendung aus mehren Zubereitungen (B1), (B2) usw. durch Vermischen erhalten wurde.

Als Oxidationsmittel kommen Persulfate, Peroxodisulfate, Chlorite, Hypochlorite und insbesondere Wasserstoffperoxid oder und/oder eines seiner festen Anlagerungsprodukte an organische oder anorganische Verbindungen in Frage.

Um eine vorzeitige, unerwünschte Reaktion der Oxidationsfarbstoffvorprodukte durch das Oxidationsmittel zu verhindern, werden Oxidationsfarbstoffvorprodukte und Oxidationsmittel selbst zweckmäßigerweise getrennt voneinander konfektioniert und erst unmittelbar vor der Anwendung in Kontakt gebracht.

In einer weiteren Ausführungsform der vorliegenden Erfindung sind daher Mittel (B) bevorzugt, welche dadurch gekennzeichnet sind, daß sie unmittelbar vor der Anwendung durch Vermischen mindestens zweier Zubereitungen hergestellt wird, wobei die mindestens zwei Zubereitungen in mindestens zwei getrennt konfektionierten Containern bereitgestellt werden und wobei ein Container ein Färbemittel (B1), welches in einem kosmetischen Träger mindestens ein Oxidationsfarbstoffvorprodukt enthält, und ein weiterer Container eine Oxidationsmittelzubereitung (B2), enthaltend mindestens ein Oxidationsmittel, enthält.

Bevorzugt enthält die Oxidationsmittelzubereitung (B2) als Oxidationsmittel Wasserstoffperoxid und/oder eines seiner festen Anlagerungsprodukte an organische oder anorganische Verbindungen, wie Harnstoff, Melamin sowie Natriumborat.

Solche Oxidationsmittelzubereitungen (B2) sind vorzugsweise wässrige, fließfähige Oxidationsmittelzubereitungen. Dabei sind bevorzugte Zubereitungen (B2) dadurch gekennzeichnet, daß die fließfähige Oxidationsmittelzubereitung (B2) - bezogen auf ihr Gewicht - 40 bis 90 Gew.-%, vorzugsweise 50 bis 85 Gew.-%, besonders bevorzugt 55 bis 80 Gew.-%, weiter bevorzugt 60 bis 77,5 Gew.-% und insbesondere 65 bis 75 Gew.-% Wasser enthält.

Bevorzugt beträgt die Menge an Oxidationsmittel im anwendungsbereiten Mittel (B) 0,5 bis 12 Gew.-%, bevorzugt 2 bis 10 Gew.-% insbesondere bevorzugt zu 3 bis 6 Gew.-% (berechnet als 100 %-iges H₂O₂), jeweils bezogen auf das anwendungsbereite Mittel (B).

In einer weiteren bevorzugten Ausführungsform ist das Mittel (B) ein Mittel zum Färben und gegebenenfalls gleichzeitigen Aufhellen keratinischer Fasern, das bevorzugt 0,5 bis 15 Gew.-%, vorzugsweise 1 bis 12,5 Gew.-%, besonders bevorzugt 1,5 bis 10 Gew.-% und insbesondere 2 bis 6 Gew.-% Wasserstoffperoxid, jeweils bezogen auf das Gesamtgewicht des anwendungsbereiten Mittels (B), enthält.

Erfindungsgemäß kann aber das Oxidationsfärbemittel auch zusammen mit einem Katalysator auf das Haar aufgebracht werden, der die Oxidation der Farbstoffvorprodukte aktiviert. Solche Katalysatoren sind z. B. bestimmte Enzyme, lodide, Chinone oder Metallionen.

Es hat sich als vorteilhaft erwiesen, wenn die erfindungsgemäßen Oxidationsmittelzubereitungen (B2) zur Stabilisierung des Wasserstoffperoxids zusätzlich mindestens einen Stabilisator oder Komplexbildner enthalten. Besonders bevorzugte Stabilisatoren sind insbesondere EDTA und EDDS, und Phosphonate, insbesondere 1-Hydroxyethan-1,1-diphosphonat (HEDP) und/oder Ethylendiamintetramethylenphosphonat (EDTMP) und/oder Diethylentriaminpentamethylenphosphonat (DTPMP) bzw. deren Natriumsalze.

Zur Erzielung einer verstärkten Aufhell- und Bleichwirkung kann das Mittel (B) weiterhin mindestens ein Peroxo-Salz enthalten. Geeignete Peroxo-Salze sind anorganische Peroxoverbindungen, bevorzugt ausgewählt aus der Gruppe Ammoniumperoxodisulfat, Alkalimetallperoxodisulfaten, Ammoniumperoxomonosulfat, Alkalimetallperoxomonosulfaten, Alkalimetallperoxodiphosphaten und Erdalkalimetallperoxiden. Besonders bevorzuge sind Peroxodisulfate, insbesondere Ammoniumperoxodisulfat, Kaliumperoxodisulfat und Natriumperoxodisulfat.

Die Persulfate sind jeweils in einer Menge von 0,5 bis 20 Gew.-%, vorzugsweise 1 bis 12,5 Gew.-%, besonders bevorzugt 2,5 bis 10 Gew.-% und insbesondere 3 bis 6 Gew.-%, bezogen auf das Gesamtgewicht des anwendungsbereiten Mittels, im Mittel (B) enthalten.

Auch das Mittel (B) kann Alkalisierungsmittel enthalten, dabei hat es sich als besonders bevorzugt erwiesen, wenn Mittel (B) einen niedrigeren pH-Wert besitzt als Mittel (A). Entsprechende erfindungsgemäße Verfahren, bei denen das Mittel (A) einen höheren pH-Wert besitzt als das Mittel (B), sind aufgrund der höheren Beständigkeiten der Färbungen bevorzugt.

Die anwendungsbereiten Färbemittel (B) können weiterhin zusätzliche Wirk-, Hilfs- und Zusatzstoffe enthalten, um die Färbeleistung zu verbessern und weitere gewünschte Eigenschaften der Mittel einzustellen.

Vorzugsweise werden die anwendungsbereiten Färbemittel als flüssige Zubereitung bereitgestellt und den Mitteln daher zusätzlich eine oberflächenaktive Substanz zugesetzt, wobei solche oberflächenaktive Substanzen je nach Anwendungsgebiet als Tenside oder als Emulgatoren bezeichnet werden: Sie sind bevorzugt aus anionischen, kationischen, zwitterionischen, amphoteren und nichtionischen Tensiden und Emulgatoren ausgewählt.

Erfindungsgemäß bevorzugte Mittel sind dadurch gekennzeichnet, daß das Mittel zusätzlich mindestens ein anionisches Tensid enthält. Bevorzugte anionische Tenside sind Fettsäuren, Alkylsulfate, Alkylethersulfate und Ethercarbonsäuren mit 10 bis 20 C-Atomen in der Alkylgruppe und bis zu 16 Glykolethergruppen im Molekül. Die anionischen Tenside werden in Anteilen von 0,1 bis 45 Gew.%, bevorzugt 1 bis 30 Gew.% und ganz besonders bevorzugt von 1 bis 15 Gew.%, bezogen auf die Gesamtmenge des anwendungsbereiten Mittels, eingesetzt.

Erfindungsgemäß bevorzugte Mittel sind dadurch gekennzeichnet, daß das Mittel zusätzlich mindestens ein zwitterionisches Tensid enthält. Bevorzugte zwitterionische Tenside sind Betaine, N-Alkyl-N,N-dimethylammonium-glycinate, N-Acyl-aminopropyl-N,N-dimethylammoniumglycinate, und 2-Alkyl-3-carboxymethyl-3-hydroxyethyl-imidazoline. Ein bevorzugtes zwitterionisches Tensid ist unter der INCI-Bezeichnung Cocamidopropyl Betaine bekannt.

Erfindungsgemäß bevorzugte Mittel sind dadurch gekennzeichnet, daß das Mittel zusätzlich mindestens ein amphoteres Tensid enthält. Bevorzugte amphotere Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren. Besonders bevorzugte amphotere Tenside sind N-Kokosalkylaminopropionat, as Kokosacylaminoethylaminopropionat und C₁₂-C₁₈-Acylsarcosin.

Weiterhin hat es sich als vorteilhaft erwiesen, wenn die Mittel weitere, nichtionogene grenzflächenaktive Stoffe, enthalten. Bevorzugte nichtionische Tenside sind beispielsweise Alkylenoxid-Anlagerungsprodukte an Fettalkohole und Fettsäuren mit jeweils 2 bis 30 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside Fettsäureester von ethoxyliertem Glycerin enthalten.

Die nichtionischen, zwitterionischen oder amphoteren Tenside werden in Anteilen von 0,1 bis 45 Gew.%, bevorzugt 1 bis 30 Gew.% und ganz besonders bevorzugt von 1 bis 15 Gew.%, bezogen auf die Gesamtmenge der anwendungsbereiten Mittel, eingesetzt.

Die anwendungsbereiten Färbemittel können weitere Hilfs- und Zusatzstoffe enthalten. So hat es sich als vorteilhaft erwiesen, wenn die Mittel mindestens ein Verdickungsmittel enthält. Bezüglich dieser Verdickungsmittel bestehen keine prinzipiellen Einschränkungen. Es können sowohl organische als auch rein anorganische Verdickungsmittel zum Einsatz kommen.

Geeignete Verdickungsmittel sind anionische, synthetische Polymere; kationische, synthetische Polymere; natürlich vorkommende Verdickungsmittel, wie nichtionische Guargums, Skleroglucangums oder Xanthangums, Gummi arabicum, Ghatti-Gummi, Karaya-Gummi, Tragant-Gummi, Carrageen-Gummi, Agar-Agar, Johannisbrotkernmehl, Pektine, Alginate, Stärke-Fraktionen und Derivate wie Amylose, Amylopektin und Dextrine, sowie Cellulosederivate, wie beispielsweise Methylcellulose, Carboxyalkylcellulosen und Hydroxyalkylcellulosen; nichtionische, synthetische Polymere, wie Polyvinylalkohol oder Polyvinylpyrrolidinon; sowie anorganische Verdickungsmittel, insbesondere Schichtsilikate wie beispielsweise Bentonit, besonders Smektite, wie Montmorillonit oder Hectorit. Auch zwitterionische Polymere können in den erfindungsgemäßen Mitteln enthalten sein.

Bevorzugte zwitterionische Polymere werden ausgewählt aus der Gruppe der
- Copolymere aus Dimethyl-diallylammoniumsalzen und Acrylsäure, z.B. Polyquaternium-22,
- Copolymere aus Dimethyl-diallylammoniumsalzen und Methacrylsäure,
- Copolymere aus N,N,N-Trimethyl-3-[(2-methyl-1-oxo-2-propen-1-yl)amino]-1-propanaminiumsalzen und Acrylsäure,
- Copolymere aus N,N,N-Trimethyl-3-[(2-methyl-1-oxo-2-propen-1-yl)amino]-1-propanaminiumsalzen und Methacrylsäure,
- Copolymere aus N,N,N-Trimethyl-2-[(2-methyl-1-oxo-2-propen-1-yl)amino]-1-ethanaminiumsalzen und Acrylsäure,
- Copolymere aus N,N,N-Trimethyl-2-[(2-methyl-1-oxo-2-propen-1-yl)amino]-1-ethanaminiumsalzen und Methacrylsäure,
- Copolymere aus N,N,N-Trimethyl-3-[(2-methyl-1-oxo-2-propen-1-yl)amino]-1-propanaminiumsalzen, Acrylsäure und Acrylamid, z.B. Polyquaternium-53
- Copolymere aus N,N,N-Trimethyl-3-[(2-methyl-1-oxo-2-propen-1-yl)amino]-1-propanaminiumsalzen, Methacrylsäure und Acrylamid,
- Copolymere aus 1-Ethenyl-3-methyl-1H-imidazoliumsalzen, 1-Ethenyl-1H-imidazol, 1-Ethenyl-2-pyrrolidinon und Methacrylsäure, z.B. Polyquaternium-86,
- Copolymere aus 1-Ethenyl-3-methyl-1H-imidazoliumsalzen, 1-Ethenyl-1H-imidazol, 1-Ethenyl-2-pyrrolidinon und Acrylsäure.

Auch Gemische der vorgenannten bevorzugten zwitterionischen Polymere (c) können in den erfindungsgemäßen Mittel enthalten sein.

Es hat sich weiterhin gezeigt, daß die erfindungsgemäße Aufgabenstellung insbesondere dann vollständig und in zufriedenstellender Weise gelöst werden kann, wenn die im erfindungsgemäßen Verfahren eingesetzten Mittel weitere ausgewählte Formulierungsbestandteile enthalten.

So hat sich herausgestellt, daß die zusätzliche Anwesenheit von bestimmten, höherkettigen Fettalkoholen das farbliche Ergebnis der erfindungsgemäßen Zusammensetzungen noch weiter verbessert. Daher ist es bevorzugt, wenn die im erfindungsgemäßen Verfahren eingesetzten Mittel zusätzlich einen oder mehrere Fettalkohole aus der Gruppe Arachylalkohol (Eisocan-1-ol), Gadoleylalkohol ((9Z)-Eicos-9-en-1-ol), Arachidonalkohol ((5Z,8Z,11Z,14Z)-Eicosa-5,8,11,14-tetraen-1-ol), Heneicosylalkohol (Heneicosan-1-ol), Behenylalkohol (Docosan-1-ol), Erucylalkohol ((13Z)-Docos-13-en-1-ol) und Brassidylalkohol ((13*E*)-Docosen-1-ol) enthalten.

Besonders geeignete Mittel enthalten einen oder mehrere höherkettige Alkohole der vorgenannten Gruppe in einer Gesamtmenge von 1,0 bis 10,0 Gew.-%, bevorzugt von 1,4 bis 8,0 Gew.-%, weiter bevorzugt von 1,8 bis 6,0 Gew.-% und besonders bevorzugt von 2,0 bis 4,0 Gew.-% - bezogen auf das Gesamtgewicht des anwendungsbereiten Mittels.

In einer weiteren bevorzugten Ausführungsform ist ein im erfindungsgemäßen Verfahren eingesetztes Mittel daher dadurch gekennzeichnet, daß es zusätzlich einen oder mehrere Fettalkohole aus der Gruppe Arachylalkohol (Eicosan-1-ol), Gadoleylalkohol ((9Z)-Eicos-9-en-1-ol), Arachidonalkohol ((5*Z*,8*Z*,11*Z*,14*Z*)-Eicosa-5,8,11,14-tetraen-1-ol), Heneicosylalkohol (Heneicosan-1-ol), Behenylalkohol (Docosan-1-ol), Erucylalkohol ((13*Z*)-Docos-13-en-1-ol) und Brassidylalkohol ((13*E*)-Docosen-1-ol) in einer Gesamtmenge von 0,1 bis 10,0 Gew.-%, bevorzugt von 1,4 bis 8,0 Gew.-%, weiter bevorzugt von 1,8 bis 6,0 Gew.-% und besonders bevorzugt von 2,0 bis 4,0 Gew.-% - bezogen auf das Gesamtgewicht des anwendungsbereiten Mittels - enthält.

Es hat sich gezeigt, daß eine Vorbehandlung bei leicht erhöhten Temperaturen die multitonalen Effekte noch lebendiger wirken läßt Erfinddungsgemäß bevorzugte Verfahren sind dadurch gekennzeichnet, daß das Mittel (A) in Schritt B) bei einer Temperatur von mindestens 40 °C einwirken gelassen wird.

## Patentansprüche

1. Verfahren zum oxidativen Färben von keratinischen Fasern, umfassend die Schritte
A) Applikation eines kosmetischen Mittels (A) auf die Fasern,
B) Einwirkenlassen des Mittels (A) für einen Zeitraum von 30 Sekunden bis 30 Minuten,
C) Applikation eines kosmetischen Mittels (B) auf die keratinischen Fasern, die noch mit dem Mittel (A) beaufschlagt sind,
D) Einwirkenlassen beider Mittel (A) und (B) für einen Zeitraum von 5 bis 45 Minuten,
E) Ausspülen der Mittel (A) und (B),
**dadurch gekennzeichnet, dass**
- das Mittel (A)
(a1) ein oder mehrere Oxidationsfarbstoffvorprodukte vom Entwicklertyp enthält,
(a2) ein oder mehrere Oxidationsfarbstoffvorprodukt vom Kupplertyp enthält,
(a3) einen pH-Wert von 8,0 bis 12,0 besitzt,
und - bezogen auf sein Gewicht - weniger als 0,01 Gew.-% Wasserstoffperoxid enthält, und
- das Mittel (B)
(b1) ein oder mehrere Oxidationsfarbstoffvorprodukte enthält,
(b2) eine oder mehrere Oxidationsmittel enthält und
(b3) einen pH-Wert von 8,0 bis 12,0 besitzt.

2. Verfahren nach Anspruch 1, **dadurch gekennzeichnet, daß** das Mittel (A) als Oxidationsfarbstoffvorprodukt vom Entwicklertyp (a1) ein oder mehrere Verbindungen aus der Gruppe p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1H-imidazol-1-yl)propyl]amin und/oder deren physiologisch verträgliche Salze in einer Gesamtmenge von 0,1 bis 3,5 Gew.-%, bevorzugt von 0,3 bis 2,8 Gew.-%, weiter bevorzugt von 0,4 bis 2,1 Gew.-% und besonders bevorzugt von 0,5 bis 1,6 Gew.-% - bezogen auf das Gesamtgewicht des Mittels (A) - enthält.

3. Verfahren nach einem der Ansprüche 1 bis 2, **dadurch gekennzeichnet, daß** das Mittel (A) als Oxidationsfarbstoffvorprodukt vom Entwicklertyp (a1) ein oder mehrere Verbindungen aus der Gruppe Bis-(2-hydroxy-5-aminophenyl)methan, 1,3-Bis-(2,5-diaminophenoxy)propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-und Amino-3-methylphenol und/oder deren physiologisch verträgliche Salzen in einer Gesamtmenge von 0,1 bis 3,5 Gew.-%, bevorzugt von 0,3 bis 2,8 Gew.-%, weiter bevorzugt von 0,4 bis 2,1 Gew.-% und besonders bevorzugt von 0,5 bis 1,6 Gew.-% - bezogen auf das Gesamtgewicht des Mittels (A) - enthält.

4. Verfahren nach einem der Ansprüche 1 bis 3, **dadurch gekennzeichnet, daß** das Mittel (A) als Oxidationsfarbstoffvorprodukt vom Entwicklertyp (a1) ein oder mehrere Verbindungen aus der Gruppe 4,5-Diamino-1-(2-hydroxyethyl)pyrazol, 2,4,5,6-Tetraaminopyrimidin, 4-Hydroxy-2,5,6-triaminopyrimidin, 2-Hydroxy-4,5,6-triaminopyrimidin, 2,3-Diamino-6,7-dihydro-1H,5H-pyrazolo[1,2-a]pyrazol-1-on und/oder deren physiologisch verträglichen Salzen in einer Gesamtmenge von 0,1 bis 3,5 Gew.-%, bevorzugt von 0,3 bis 2,8 Gew.-%, weiter bevorzugt von 0,4 bis 2,1 Gew.-% und besonders bevorzugt von 0,5 bis 1,6 Gew.-% - bezogen auf das Gesamtgewicht des Mittels (A) - enthält.

5. Verfahren nach einem der Ansprüche 1 bis 4, **dadurch gekennzeichnet, daß** das Mittel (A) als Oxidationsfarbstoffvorpdodukt vom Kupplertyp (a2) ein oder mehrere Verbindungen aus der Gruppe 3-Aminophenol, 5-Amino-2-methylphenol, 3-Amino-2-chlor-6-methylphenol, 2-Hydroxy-4-aminophenoxyethanol, 5-Amino-4-chlor-2-methylphenol, 5-(2-Hydroxyethyl)-amino-2-methylphenol, 2,4-Dichlor-3-aminophenol, 2-Aminophenol und/oder deren physiologisch verträgliche Salze in einer Gesamtmenge von 0,1 bis 3,5 Gew.-%, bevorzugt von 0,3 bis 2,8 Gew.-%, weiter bevorzugt von 0,4 bis 2,1 Gew.-% und besonders bevorzugt von 0,5 bis 1,6 Gew.-% - bezogen auf das Gesamtgewicht des Mittels (A) - enthält.

6. Verfahren nach einem der Ansprüche 1 bis 5, **dadurch gekennzeichnet, daß** das Mittel (A) als Oxidationsfarbstoffvorpdodukt vom Kupplertyp (a2) ein oder mehrere Verbindungen aus der Gruppe 3-Phenylendiamin, 2-(2,4-Diaminophenoxy)ethanol, 1,3-Bis(2,4-diaminophenoxy)propan, 1-Methoxy-2-amino-4-(2-hydroxyethylamino)benzol, 1,3-Bis(2,4-diaminophenyl)propan, 2,6-Bis(2'-hydroxyethylamino)-1-methylbenzol und/oder deren physiologisch verträgliche Salze in einer Gesamtmenge von 0,1 bis 3,5 Gew.-%, bevorzugt von 0,3 bis 2,8 Gew.-%, weiter bevorzugt von 0,4 bis 2,1 Gew.-% und besonders bevorzugt von 0,5 bis 1,6 Gew.-% - bezogen auf das Gesamtgewicht des Mittels (A) - enthält.

7. Verfahren nach einem der Ansprüche 1 bis 6, **dadurch gekennzeichnet, daß** das Mittel (A) als Oxidationsfarbstoffvorpdodukt vom Kupplertyp (a2) ein oder mehrere Verbindungen aus der Gruppe Resorcin, 2-Methylresorcin und/oder 4-Chlorresorcin in einer Gesamtmenge von 0,1 bis 3,5 Gew.-%, bevorzugt von 0,3 bis 2,8 Gew.-%, weiter bevorzugt von 0,4 bis 2,1 Gew.-% und besonders bevorzugt von 0,5 bis 1,6 Gew.-% - bezogen auf das Gesamtgewicht des Mittels (A) - enthält.

8. Verfahren nach einem der Ansprüche 1 bis 7, **dadurch gekennzeichnet, daß** das Mittel (A) als Oxidationsfarbstoffvorpdodukt vom Kupplertyp (a2) ein oder mehrere Verbindungen aus der Gruppe 2-Amino-3-hydroxypyridin, 3-Amino-2-methylamino-6-methoxypyridin, 2,6-Dihydroxy-3,4-dimethylpyridin, 3,5-Diamino-2,6-dimethoxypyridin, 1-Phenyl-3-methylpyrazol-5-on und/oder deren physiologisch verträgliche Salze in einer Gesamtmenge von 0,1 bis 3,5 Gew.-%, bevorzugt von 0,3 bis 2,8 Gew.-%, weiter bevorzugt von 0,4 bis 2,1 Gew.-% und besonders bevorzugt von 0,5 bis 1,6 Gew.-% - bezogen auf das Gesamtgewicht des Mittels (A) - enthält.

9. Verfahren nach einem der Ansprüche 1 bis 8, **dadurch gekennzeichnet, daß** das Mittel (A) als Oxidationsfarbstoffvorpdodukt vom Kupplertyp (a2) mindestens eine der folgenden Kombinationen enthält: Resorcin/3-Aminophenol; 2-Methylresorcin/3-Aminophenol; 4-Chlorresorcin/3-Aminophenol; Resorcin/5-Amino-2-methylphenol; 2-Methylresorcin/5-Amino-2-methylphenol; 4-Chlorresorcin/5-Amino-2-methylphenol; Resorcin/2-Hydroxy-4-aminophenoxyethanol; 2-Methylresorcin/2-Hydroxy-4-aminophenoxyethanol; 4-Chlorresorcin/2-Hydroxy-4-aminophenoxyethanol; Resorcin/2-Amino-3-hydroxypyridin; 2-Methylresorcin/2-Amino-3-hydroxypyridine; 4-Chlorresorcin/2-Amino-3-hydroxypyridine; Resorcin/3-Amino-2-methylamino-6-methoxypyridin; 2-Methylresorcin/3-Amino-2-methylamino-6-methoxypyridin; 4-Chlorresorcin/3-Amino-2-methylamino-6-methoxypyridin; Resorcin/2,6-Dihydroxy-3,4-dimethylpyridin; 2-Methylresorcin/2,6-Dihydroxy-3,4-dimethylpyridin und/oder 4-Chlorresorcin/2,6-Dihydroxy-3,4-dimethylpyridin und/oder deren physiologisch verträglichen Salze.

10. Verfahren nach einem der Ansprüche 1 bis 9, **dadurch gekennzeichnet, daß** das Mittel (A),
einen pH-Wert (a3) von 8,5 bis 11,5, bevorzugt von 8,8 bis 11,0, weiter bevorzugt von 9,0 bis 10,8 und besonders bevorzugt von 9,2 bis 10,5 besitzt.

11. Verfahren nach einem der Ansprüche 1 bis 10, **dadurch gekennzeichnet, daß** das Mittel (A), zusätzlich ein oder mehrere Alkalisierungsmittel aus der Gruppe Natriumhydroxid, Kaliumhydroxid, Ammoniak, Monoethanolamin und 2-Amino-2-methylpropanol in einer Gesamtmenge von 0,3 bis 4,5 Gew.-%, bevorzugt 0,5 bis 3,5 Gew.-%, weiter bevorzugt von 0,7 bis 2,5 Gew.-% und besonders bevorzugt von 0,9 bis 1,5 Gew.-% - bezogen auf das Gesamtgewicht des Mittels (A) - enthält.

12. Verfahren nach einem der Ansprüche 1 bis 11, **dadurch gekennzeichnet, daß** das Mittel (A) in Schritt B) für einen Zeitraum von 30 Sekunden bis 15 Minuten, bevorzugt von 30 Sekunden bis 10 Minuten und besonders bevorzugt von 30 Sekunden bis 5 Minuten auf den Fasern einwirken gelassen wird.

13. Verfahren nach einem der Ansprüche 1 bis 12, **dadurch gekennzeichnet, daß** das Mittel (A), zusätzlich mindestens ein Verdickungsmittel und/oder mindestens einen Gelbildner enthält.

14. Verfahren nach einem der Ansprüche 1 bis 13, **dadurch gekennzeichnet, daß** das Mittel (A) und das Mittel (B) die gleichen Oxidationsfarbstoffvorprodukte enthalten.

15. Verfahren nach einem der Ansprüche 1 bis 14, **dadurch gekennzeichnet, daß** die Mittel (A) und (B) in Schritt D) für einen Zeitraum von 5 bis 30 Minuten, bevorzugt von 5 bis 20 Minuten, besonders bevorzugt von 5 bis 15 Minuten einwirken gelassen werden.

## Claims

1. A method for oxidatively dyeing keratin fibers, comprising the steps of
A) applying a cosmetic agent (A) to the fibers,
B) allowing the agent (A) to act for a period of 30 seconds to 30 minutes,
C) applying a cosmetic agent (B) to the keratin fibers, which are still subjected to agent (A),
D) allowing the two agents (A) and (B) to act for a period of 5 to 45 minutes,
E) rinsing out agents (A) and (B), **characterized in that**
- agent (A)
(a1) contains one or more developer-type oxidation dye precursors,
(a2) contains one or more coupler-type oxidation dye precursors,
(a3) has a pH of 8.0 to 12.0,
**characterized in that** the agent (A) contains less than 0.01% by weight of hydrogen peroxide, based on its weight, and
- agent (B)
(b1) contains one or more oxidation dye precursors,
(b2) contains one or more oxidizing agents and
(b3) has a pH of 8.0 to 12.0.

2. The method according to claim 1, **characterized in that** agent (A) contains, as the developer-type oxidation dye precursor (a1), one or more compounds from the group comprising p-Phenylenediamine, p-Toluenediamine, 2-(2-hydroxyethyl)-p-Phenylenediamine, 2-(1,2-dihydroxyethyl)-p-Phenylenediamine, N,N-bis-(2-hydroxyethyl)-p-Phenylenediamine, 2-Methoxymethyl-p-Phenylenediamine, N-(4-Amino-3-methylphenyl)-N-[3-(1H-lmidazol-1-yl)propyl]amine and/or the physiologically acceptable salts thereof in a total amount of 0.1 to 3.5 wt.%, preferably of 0.3 to 2.8 wt.%, more preferably of 0.4 to 2.1 wt.% and particularly preferably of 0.5 to 1.6 wt.%, based on the total weight of agent (A).

3. The method according to one of claims 1 to 2, **characterized in that** agent (A) contains, as the developer-type oxidation dye precursor (a1), one or more compounds from the group comprising bis-(2-hydroxy-5-aminophenyl)methane, 1,3-bis-(2,5-diaminophenoxy)propan-2-ol, N,N'-bis-(4-aminophenyl)-1,4-Diazacycloheptane, 1,10-bis-(2,5-diaminophenyl)-1,4,7,10-tetraoxadecane, p-aminophenol, 4- and amino-3-methylphenol and/or the physiologically acceptable salts thereof in a total amount of 0.1 to 3.5 wt.%, preferably of 0.3 to 2.8 wt.%, more preferably of 0.4 to 2.1 wt.% and particularly preferably of 0.5 to 1.6 wt.%, based on the total weight of agent (A).

4. The method according to one of claims 1 to 3, **characterized in that** agent (A) contains, as the developer-type oxidation dye precursor (a1), one or more compounds from the group comprising 4,5-diamino-1-(2-hydroxyethyl)pyrazole, 2,4,5,6-tetraaminopyrimidine, 4-hydroxy-2,5,6-triaminopyrimidine, 2-hydroxy-4,5,6-triaminopyrimidine, 2,3-diamino-6,7-dihydro-1H,5H-pyrazolo[1,2-a]pyrazol-1-one and/or the physiologically acceptable salts thereof in a total amount of 0.1 to 3.5 wt.%, preferably of 0.3 to 2.8 wt.%, more preferably of 0.4 to 2.1 wt.% and particularly preferably of 0.5 to 1.6 wt.%, based on the total weight of agent (A).

5. The method according to one of claims 1 to 4, **characterized in that** agent (A) contains, as the coupler-type oxidation dye precursor (a2), one or more compounds from the group comprising 3-aminophenol, 5-amino-2-methylphenol, 3-amino-2-chloro-6-methylphenol, 2-hydroxy-4-aminophenoxyethanol, 5-amino-4-chloro-2-methylphenol, 5-(2-hydroxyethyl)-amino-2-methyl-phenol, 2,4-dichloro-3-aminophenol, 2-aminophenol and/or the physiologically acceptable salts thereof in a total amount of 0.1 to 3.5 wt.%, preferably of 0.3 to 2.8 wt.%, more preferably of 0.4 to 2.1 wt.% and particularly preferably of 0.5 to 1.6 wt.%, based on the total weight of agent (A).

6. The method according to one of claims 1 to 5, **characterized in that** agent (A) contains, as the coupler-type oxidation dye precursor (a2), one or more compounds from the group comprising 3-Phenylenediamine, 2-(2,4-diaminophenoxy)ethanol, 1,3-bis(2,4-diaminophenoxy)propane, 1 -methoxy-2-amino-4-(2-hydroxyethylamino)benzene, 1,3-bis(2,4-diamino-phenyl)propane, 2,6-bis(2'-hydroxyethylamino)-1-methylbenzene and/or the physiologically acceptable salts thereof in a total amount of 0.1 to 3.5 wt.%, preferably of 0.3 to 2.8 wt.%, more preferably of 0.4 to 2.1 wt.% and particularly preferably of 0.5 to 1.6 wt.%, based on the total weight of agent (A).

7. The method according to one of claims 1 to 6, **characterized in that** agent (A) contains, as the coupler-type oxidation dye precursor (a2), one or more compounds from the group comprising resorcinol, 2-methylresorcinol and/or 4-chlororesorcinol in a total amount of 0.1 to 3.5 wt.%, preferably of 0.3 to 2.8 wt.%, more preferably of 0.4 to 2.1 wt.% and particularly preferably of 0.5 to 1.6 wt.%, based on the total weight of agent (A).

8. The method according to one of claims 1 to 7, **characterized in that** agent (A) contains, as the coupler-type oxidation dye precursor (a2), one or more compounds from the group comprising 2-amino-3-hydroxypyridine, 3-amino-2-methylamino-6-methoxypyridine, 2,6-dihydroxy-3,4-dimethylpyridine, 3,5-diamino-2,6-dimethoxypyridine, 1-phenyl-3-methylpyrazol-5-one and/or the physiologically acceptable salts thereof in a total amount of 0.1 to 3.5 wt.%, preferably of 0.3 to 2.8 wt.%, more preferably of 0.4 to 2.1 wt.% and particularly preferably of 0.5 to 1.6 wt.%, based on the total weight of agent (A).

9. The method according to one of claims 1 to 8, **characterized in that** agent (A) contains, as the coupler-type oxidation dye precursor (a2), at least one of the following combinations: resorcinol/3-aminophenol; 2-methylresorcinol/3-aminophenol; 4-chlororesorcinol/3-aminophenol; resorcinol/5-amino-2-methylphenol; 2-methylresorcinol/5-amino-2-methylphenol; 4-chlororesorcinol/5-amino-2-methylphenol; resorcinol/2-hydroxy-4-aminophenoxyethanol; 2-methylresorcinol/2-hydroxy-4-aminophenoxyethanol; 4-chlororesorcinol/2-hydroxy-4-aminophenoxyethanol; resorcinol/2-amino-3-hydroxypyridine; 2-methylresorcinol/2-amino-3-hydroxy-pyridine; 4-chlororesorcinol/2-amino-3-hydroxypyridine; resorcinol/3-amino-2-methylamino-6-methoxypyridine; 2-methylresorcinol/3-amino-2-methylamino-6-methoxypyridine; 4-chlororesorcinol/3-amino-2-methylamino-6-methoxypyridine; resorcinol/2,6-dihydroxy-3,4-dimethylpyridine; 2-methylresorcinol/2,6-dihydroxy-3,4-dimethylpyridine and/or 4-chlororesorcinol/2,6-dihydroxy-3,4-dimethylpyridine and/or the physiologically acceptable salts thereof.

10. The method according to one of claims 1 to 9, **characterized in that** agent (A) has a pH (a3) of 8.5 to 11.5, preferably of 8.8 to 11.0, more preferably of 9.0 to 10.8 and particularly preferably of 9.2 to 10.5.

11. The method according to one of claims 1 to 10, **characterized in that** agent (A) also contains one or more alkalizing agents from the group comprising sodium hydroxide, potassium hydroxide, ammonia, monoethanolamine and 2-amino-2-methylpropanol in a total amount of 0.3 to 4.5 wt.%, preferably 0.5 to 3.5 wt.%, more preferably 0.7 to 2.5 wt.% and particularly preferably 0.9 to 1.5 wt.%, based on the total weight of agent (A).

12. The method according to one of claims 1 to 11, **characterized in that** in step B), agent (A) is allowed to act on the fibers for a period of 30 seconds to 15 minutes, preferably of 30 seconds to 10 minutes and particularly preferably of 30 seconds to 5 minutes.

13. The method according to one of claims 1 to 12, **characterized in that** agent (A) also contains at least one thickening agent and/or at least one gelling agent.

14. The method according to one of claims 1 to 13, **characterized in that** agent (A) and agent (B) contain the same oxidation dye precursors.

15. The method according to one of claims 1 to 14, **characterized in that** in step D), agents (A) and (B) are allowed to act for a period of 5 to 30 minutes, preferably of 5 to 20 minutes, particularly preferably of 5 to 15 minutes.

## Revendications

1. Procédé de coloration oxydative de fibres kératiniques, comprenant les étapes consistant à
A) appliquer un produit cosmétique (A) sur les fibres ;
B) laisser agir le produit (A) pendant une période de 30 secondes à 30 minutes ;
C) appliquer un produit cosmétique (B) sur les fibres kératiniques sur lesquelles le produit (A) n'a pas été encore appliqué ;
D) laisser agir les deux produits (A) et (B) pendant une période de 5 à 45 minutes ;
E) rincer les produits (A) et (B), **caractérisé en ce que**
- le produit (A)
(a1) contient un ou plusieurs précurseurs de coloration par oxydation de type bases,
(a2) contient un ou plusieurs précurseurs de coloration par oxydation de type coupleurs,
(a3) possède un taux de pH compris entre 8,0 et 12,0 ;
**caractérisé en ce que** l'agent (A) contient - par rapport à son poids - moins de 0,01 % en poids de peroxyde d'hydrogène, et
- le produit (B)
(b1) contient un ou plusieurs précurseurs de coloration par oxydation,
(b2) contient un ou plusieurs précurseurs de coloration par oxydation et possède (b3) un taux de pH compris entre 8,0 et 12,0.

2. Procédé, selon la revendication 1, **caractérisé en ce que** le produit (A), en tant que précurseur de coloration par oxydation de type base contient (a1) un ou plusieurs composants appartenant au groupe p-phénylènediamine, p-toluènediamine, 2-(2-hydroxyethyl)-p-phénylènediamine, 2-(1,2-dihydroxyethyl)-p-phénylènediamine, N,N-Bis-(2-hydroxyethyl)-p-phénylènediamine, 2-methoxymethyl-p-phénylènediamine, N-(4-amino-3-methylphenyl)-N-[3-(1H-imidazol-1-yl)propyl]amine et/ou leurs sels physiologiquement acceptables en une quantité totale comprise entre 0,1 et 3,5 % en poids, de préférence de 0,3 à 2,8 % en poids, plus préférablement de 0,4 à 2,1 % en poids et encore plus préférablement de 0,5 à 1,6 % en poids par rapport au poids total du produit (A).

3. Procédé, selon l'une des revendications 1 à 2, **caractérisé en ce que** le produit (A), en tant que précurseur de coloration par oxydation de type coupleur contient (a1) un ou plusieurs composants appartenant au groupe Bis-(2-hydroxy-5-aminophenyl)méthane, 1,3-Bis-(2,5-diaminophenoxy)propan-2-ol, N,N'-Bis-(4-aminophenyl)-1,4-diazacycloheptane, 1,10-Bis-(2,5-diaminophenyl)-1,4,7,10-tétraoxadécane, p-aminophénol, 4-et amino-3-méthylphénol et/ou leurs sels physiologiquement acceptables en une quantité totale comprise entre 0,1 et 3,5 % en poids, de préférence de 0,3 à 2,8 % en poids, plus préférablement de 0,4 à 2,1 % en poids et encore plus préférablement de 0,5 à 1,6 % en poids par rapport au poids total du produit (A).

4. Procédé, selon l'une des revendications 1 à 3, **caractérisé en ce que** le produit (A), en tant que précurseur de coloration par oxydation de type base contient (a1) un ou plusieurs composants appartenant au groupe 4,5-diamino-1-(2-hydroxyethyl)pyrazole, 2,4,5,6-tétraaminopyrimidine, 4-hydroxy-2,5,6-triaminopyrimidine, 2-hydroxy-4,5,6-triaminopyrimidine, 2,3-diamino-6,7-dihydro-1H,5H-pyrazolo[1,2-a]pyrazol-1-on et/ou leurs sels physiologiquement acceptables en une quantité totale comprise entre 0,1 et 3,5 % en poids, de préférence de 0,3 à 2,8 % en poids, plus préférablement de 0,4 à 2,1 % en poids et encore plus préférablement de 0,5 à 1,6 % en poids par rapport au poids total du produit (A).

5. Procédé, selon l'une des revendications 1 à 4, **caractérisé en ce que** le produit (A), en tant que précurseur de coloration par oxydation de type coupleur contient (a2) un ou plusieurs composants appartenant au groupe 3-aminophénol, 5-amino-2-méthylphénol, 3-amino-2-chlor-6-méthylphénol, 2-hydroxy-4-aminophénoxyéthanol, 5-amino-4-chlor-2-méthylphénol, 5-(2-hydroxyethyl)-amino-2-méthylphénol, 2,4-dichlor-3-aminophénol, 2-aminophénol et/ou leurs sels physiologiquement acceptables en une quantité totale comprise entre 0,1 et 3,5 % en poids, de préférence de 0,3 à 2,8 % en poids, plus préférablement de 0,4 à 2,1 % en poids et encore plus préférablement de 0,5 à 1,6 % en poids par rapport au poids total du produit (A).

6. Procédé, selon l'une des revendications 1 à 5, **caractérisé en ce que** le produit (A), en tant que précurseur de coloration par oxydation de type coupleur contient (a2) un ou plusieurs composants appartenant au groupe 3-phénylènediamine, 2-(2,4-diaminophenoxy)éthanol, 1,3-Bis(2,4-diaminophenoxy)propane, 1-methoxy-2-amino-4-(2-hydroxyethylamino)benzène, 1,3-Bis(2,4-diaminophenyl)propane, 2,6-Bis(2'-hydroxyethylamino)-1-méthylbenzène et/ou leurs sels physiologiquement acceptables en une quantité totale comprise entre 0,1 et 3,5 % en poids, de préférence de 0,3 à 2,8 % en poids, plus préférablement de 0,4 à 2,1 % en poids et encore plus préférablement de 0,5 à 1,6 % en poids par rapport au poids total du produit (A).

7. Procédé, selon l'une des revendications 1 à 6, **caractérisé en ce que** le produit (A), en tant que précurseur de coloration par oxydation de type coupleur contient (a2) un ou plusieurs composants appartenant au groupe résorcinol, 2-méthylrésorcinol et/ou 4-chlororésorcinol en une quantité totale comprise entre 0,1 et 3,5 % en poids, de préférence de 0,3 à 2,8 % en poids, plus préférablement de 0,4 à 2,1 % en poids et encore plus préférablement de 0,5 à 1,6 % en poids par rapport au poids total du produit (A).

8. Procédé, selon l'une des revendications 1 à 7, **caractérisé en ce que** le produit (A), en tant que précurseur de coloration par oxydation de type coupleur contient (a2) un ou plusieurs composants appartenant au groupe 2-amino-3-hydroxypyridine, 3-amino-2-methylamino-6-méthoxypyridine, 2,6-dihydroxy-3,4-diméthylpyridine, 3,5-diamino-2,6-diméthoxypyridine, 1-phenyl-3-methylpyrazol-5-on et/ou leurs sels physiologiquement acceptables en une quantité totale comprise entre 0,1 et 3,5 % en poids, de préférence de 0,3 à 2,8 % en poids, plus préférablement de 0,4 à 2,1 % en poids et encore plus préférablement de 0,5 à 1,6 % en poids par rapport au poids total du produit (A).

9. Procédé, selon l'une des revendications 1 à 8, **caractérisé en ce que** le produit (A), en tant que précurseur de coloration par oxydation de type coupleur contient (a2) au moins l'une des associations suivantes : Résorcinol/3-aminophénol ; 2-méthylrésorcinol/3-aminophénol ; 4-chlororésorcinol/3-aminophénol ; résorcinol/5-amino-2-méthylphénol ; 2-méthylrésorcinol/5-aminométhylphénol ; 4-chlororésorcinol/5-amino-2-méthylphénol ; résorcinol/2-hydroxy-4-aminophénoxyéthanol ; 2-méthylrésorcinol/2-hydroxy-4-aminophénoxyéthanol ; 4-chlororésorcinol/2-hydroxy-4-aminophénoxyéthanol ; résorcinol/2-amino-3-hydroxypyridine ; 2-méthylrésorcinol/2-amino-3-hydroxypyridine ; 4-chlororésorcinol/2-amino-3-hydroxypyridine ; résorcinol/3-amino-2-methylamino-6-méthoxypyridine ; 2-méthylrésorcinol/3-amino-2-methylamino-6-méthoxypyridine ; 4-chlororésorcinol/3-amino-2-methylamino-6-méthoxypyridine ; résorcinol/2,6-dihydroxy-3,4-diméthylpyridine ; 2-méthylrésorcinol/2,6-dihydroxy-3,4-v et/ou 4-chlororésorcinol/2,6-dihydroxy-3,4-v et/ou leurs sels physiologiquement acceptables.

10. Procédé, selon l'une des revendications 1 à 9, **caractérisé en ce que** le produit (A) possède un taux pH (a3) compris entre 8,5 et 11,5, de préférence de 8,8 à 11,0, plus préférablement de 9,0 à 10,8 et encore plus préférablement de 9,2 à 10,5.

11. Procédé, selon l'une des revendications 1 à 10, **caractérisé en ce que** le produit (A) contient également un ou plusieurs agents alcalinisants appartenant au groupe hydroxyde de sodium, hydroxyde de potassium, ammoniac, monoéthanolamine et 2-amino-2-méthylpropanol en une quantité totale comprise entre 0,3 et 4,5 % en poids, de préférence de 0,5 à 3,5 % en poids, plus préférablement de 0,7 à 2,5 % en poids et encore plus préférablement de 0,9 à 1,5 % en poids par rapport au poids total du produit (A).

12. Procédé, selon l'une des revendications 1 à 11, **caractérisé en ce que**, dans l'étape B), on laisse agir le produit (A) sur les fibres pendant une période de 30 secondes à 15 minutes, de préférence de 30 secondes à 10 minutes et encore plus préférablement de 30 secondes à 5 minutes.

13. Procédé, selon l'une des revendications 1 à 12, **caractérisé en ce que** le produit (A) contient au moins un agent épaississant et/ou au moins agent gélifiant.

14. Procédé, selon l'une des revendications 1 à 13, **caractérisé en ce que** le produit (A) et le produit (B) contiennent les mêmes précurseurs de coloration par oxydation.

15. Procédé, selon l'une des revendications 1 à 14, **caractérisé en ce que**, dans l'étape D), on laisse agir les produits (A) et (B) pendant une période de 5 à 30 minutes, de préférence de 5 à 20 minutes, encore plus préférablement de 5 à 15 minutes.
